# EUROPÄISCHE PATENTSCHRIFT

(11) **EP 2 229 407 B1**
(45) Veröffentlichungstag und Bekanntmachung des Hinweises auf die Patenterteilung: **16.11.2016**
(21) Anmeldenummer: 09701358.5
(22) Anmeldetag: 06.01.2009
(51) Int. Cl.: C07K 14/62

(54) **NEUE INSULINDERIVATE MIT EXTREM VERZÖGERTEM ZEIT- / WIRKUNGSPROFIL**
NOVEL INSULIN DERIVATIVES HAVING AN EXTREMELY DELAYED TIME-ACTION PROFILE
NOUVEAUX DÉRIVÉS D'INSULINE À PROFIL TEMPOREL/D'ACTION EXTRÊMEMENT RETARDÉ

(30) Priorität: 09.01.2008 DE 102008003568; 14.04.2008 US 44659 P; 24.05.2008 DE 102008025008
(43) Veröffentlichungstag der Anmeldung: 22.09.2010
(73) Patentinhaber: Sanofi-Aventis Deutschland GmbH, 65929 Frankfurt am Main (DE)
(72) Erfinder: HABERMANN, Paul, 65926 Frankfurt am Main (DE); SEIPKE, Gerhard, 65926 Frankfurt am Main (DE); KURRLE, Roland, 65926 Frankfurt am Main (DE); MÜLLER, Günter, 65926 Frankfurt am Main (DE); SOMMERFELD, Mark, 65926 Frankfurt am Main (DE); TENNAGELS, Norbert, 65926 Frankfurt am Main (DE); TSCHANK, Georg, 65926 Frankfurt am Main (DE); WERNER, Ulrich, 65926 Frankfurt am Main (DE)
(74) Vertreter: Weickmann & Weickmann PartmbB
(86) Internationale Anmeldenummer: PCT/EP2009/000017
(87) Internationale Veröffentlichungsnummer: WO 2009/087081

(56) Entgegenhaltungen:
- WO-A-01/25278
- WO-A-89/10937
- WO-A-02/079250
- WO-A-03/053339
- WO-A-2007/081824
- US-A- 6 100 376
- MARKUSSEN J ET AL: "SOLUBLE, PROLONGED-ACTING INSULIN DERIVATIVES. I. DEGREE OF PROTRACTION AND CRYSTALLIZABILITY OF INSULINS SUBSTITUTED IN THE TERMINI OF THE B-CHAIN", PROTEIN ENGINEERING, OXFORD UNIVERSITY PRESS, SURREY, GB, vol. 1, no. 3, 1 June 1987 (1987-06-01), pages 205-213, XP000000187, ISSN: 0269-2139

## Beschreibung

Die Erfindung betrifft neue Insulinanaloga mit basalem Zeit- / Wirkungsprofil, ihre Herstellung und Verwendung.

Über die letzten Jahre hat die Zahl der Erkrankungen an Diabetes in einem geradezu epidemischen Ausmaß zugenommen. Aufgrund der Erkrankung kann es zu einer gravierenden Verkürzung der Lebenserwartung kommen. Menschen mit Diabetes müssen ihrem Körper oft Insulin von außen zuführen. Es ist sinnvoll, die Behandlung mit Insulin zu optimieren. Mittlerweile gibt es unterschiedliche Insuline mit spezifischen pharmakologischen Eigenschaften zur Behandlung. Praktischerweise werden die unterschiedlichen Insuline nach ihrer Wirkdauer unterschieden in kurzwirksame Insuline, schnellwirkende Insuline, langwirksame Insuline und Mischinsuline. Synonym verwendete Bezeichnungen für langwirksame Insuline sind Verzögerungsinsulin, Depotinsulin oder auch Basalinsulin. Die Wirkstoffe vieler dieser Insulinpräparate sind sogenannte Insulinanaloga, die vom humanen Insulin abgeleitet worden sind durch Substitution, Deletion und / oder Addition einer oder mehrerer Aminosäuren. Die Begriffe "Insulinanaloga" und "Insuline" werden hier synonym verwendet.

Das Konzept der intensivierten Insulintherapie versucht das Gesundheitsrisiko abzumindern, indem eine stabile Kontrolle des Blutzuckerspiegels durch frühe Gabe von Basalinsulinen angestrebt wird. Ein Beispiel für ein gängiges Basalinsulin ist das Medikament Lantus^{®} (Wirkstoff: Insulin Glargin = Gly (A21), Arg (B31), Arg (B32) Humaninsulin). Generell gilt es, bei der Entwicklung neuer, verbesserter Basalinsuline die Zahl hypoglykämischer Ereignisse zu minimieren. Ein ideales Basalinsulin wirkt dabei sicher in jedem Patienten mindestens 24 Stunden. Idealerweise setzt die Insulinwirkung verzögert und mit einem möglichst flachen Zeit-/ Wirkungsprofil ein, so dass die Gefahr einer kurzfristigen Unterzuckerung deutlich minimiert ist und die Applikation sogar ohne vorherige Einnahme von Nahrungsmitteln erfolgen kann. Eine gute Versorgung mit Basalinsulin ist dann gegeben, wenn die Insulinwirkung möglichst lange gleichbleibend anhält, d.h. der

Körper mit einer konstanten Menge Insulin versorgt wird. Damit ist die Gefahr hypoglykämischer Ereignisse gering und eine patienten- und tagesspezifische Variabilität minimiert. Das pharmakokinetische Profil eines idealen Basalinsulins sollte also durch einen verzögerten Wirkeintritt und durch eine verzögerte, d.h. lang anhaltende und gleichmäßige Wirkung gekennzeichnet sein.

Jedoch zeigt - trotz der bereits erreichten therapeutischen Vorteile - keines der bisher beschriebenen Verzögerungsinsuline die pharmakokinetischen Eigenschaften eines idealen Basalinsulins. Wünschenswert sind Insuline, die ein solch flaches und lang andauerndes Zeit- / Wirkungsprofil haben, dass die Gefahr hypoglykämischer Ereignisse und der tagesabhängigen Varianz im Patienten weiter minimiert ist und die Wirkdauer weiter verzögert ist, so dass unter Umständen nicht mehr täglich Insulin verabreicht werden muss. Dies würde eine vereinfachte Behandlung von Diabetikern ermöglichen, insbesondere von älteren und pflegebedürftigen Diabetikern, die sich Insulin nicht mehr selber injizieren können und wäre somit auch von großem volkswirtschaftlichem Nutzen. In der frühen Phase des Diabetes Typ 2 wären solche Basalinsuline zudem nützlich. Kliniker berichten, dass die bei vielen Menschen vorhandene Phobie vor Spritzen sie davor zurückschrecken lässt, rechtzeitig mit der Insulintherapie zu beginnen. Als Konsequenz ergibt sich eine schlechte Blutzuckereinstellung, die diabetische Spätfolgen nach sich zieht. Ein Basalinsulin, das die Anzahl der durch Spritzen erfolgten Insulingaben vermindert, könnte bewirken, dass Patienten leichter die Insulintherapie annehmen.

US 6,100,376 offenbart Insulinderivate mit einem isoelektrischen Punkt zwischen 5 und 8.5 mit einer erhöhten Stabilität in leicht saurem wässrigem Medium. US 6,100,376 beschreibt die C-terminale Modifikation Arg-Arg-OH an der B-Kette.

WO 2007/081824 offenbart Insulinanaloga mit Histidinresten gegen Fibrillierung an den Positionen A4, A8 und B1.

WO 89/10937 betrifft die Stabilisierung von Insulinanaloga gegen chemische Veränderungen, wie z.B. Desamidierung oder Di- bzw. Polymerisierung. WO 89/10937 offenbart Modifikationen an den Positionen A15, A18, A21, B3, B4 und B10.

Markussen et al. (Protein Engineering, 1987,1:205-213) offenbaren Insulinanaloga mit verlängerter Wirkung und beschreiben Substitutionen an den Positionen B-1, B1, B29, B30, B31 und B32. Substituiert wird mit den Aminosäuren Thr, Lys, Asp, Arg und Phe.

Kohn et al. (Peptides 28 (2007) 935-948) beschreiben, dass man die Optimierung der Pharmakodynamik von Insulin dadurch erreichen kann, dass man Insulinanaloga herstellt, deren isoelektrischer Punkt (pl) durch Addition von Lysin oder Arginin an das B- Kettenende oder den N-Terminus der A und B- Kette in Richtung des alkalischen Bereichs verschoben ist, verglichen mit dem isoelektrischen Punkt von Humaninsulin (pl = 5,6), so dass die Löslichkeit unter physiologischen Bedingungen verringert ist und sich ein verlängertes Zeit- / Wirkungsprofil ergibt. Verbindung 18 aus Kohn et al. (Arg (A0), Gly (A21), Arg (B31), Arg (B32) Humaninsulin (pl experimentell bestimmt = 7,3; pl berechnet = 7,58) wird dabei als beste Verbindung im Sinne des Konzeptes dargestellt. Das hauptsächliche Ziel bei der Gestaltung neuer Insulinanaloga sehen Kohn et al. daher in der Addition von positiv geladenen Aminosäuren zur Aminosäuresequenz von Humaninsulin zwecks Erhöhung des isoelektrischen Punktes von pl = 5,6 in den neutralen Bereich.

Diesem Ziel bei der Gestaltung neuer Insulinanaloga entgegengerichtet ist die Substitution neutraler Aminosäuren in humanem Insulin durch saure Aminosäuren und / oder die Addition von sauren Aminosäuren, da solch eine Substitution und / oder Additionen den Effekt der Einführung positiv geladener Aminosäuren zumindest teilweise aufhebt. Es wurde nun aber überraschend gefunden, dass solche Insulinanaloga zu dem beschriebenen wünschenswerten basalen Zeit- / Wirkungsprofil führen, die durch die Merkmale charakterisiert sind, dass
- das B- Kettenende aus einem amidierten basischen Aminosäurerest wie Lysin bzw. Argininamid besteht, d.h. bei dem amidierten basischen Aminosäurerest am B-Kettenende liegt die Carboxylgruppe der endständigen Aminosäure in ihrer amidierten Form vor, und
- der N-terminale Aminosäurerest der Insulin A-Kette ein Lysin- oder Argininrest ist, und
- die Aminosäureposition A8 durch einen Histidinrest besetzt wird, und
- die Aminosäureposition A21 durch einen Glycinrest besetzt wird, und
- zwei Substitutionen neutraler Aminosäuren durch saure Aminosäuren, zwei Additionen negativ geladener Aminosäurereste oder je eine solche Substitution und eine solche Addition jeweils in den Positionen A5, A15, A18, B-1, B0, B1, B2, B3 und B4 erfolgt sind.

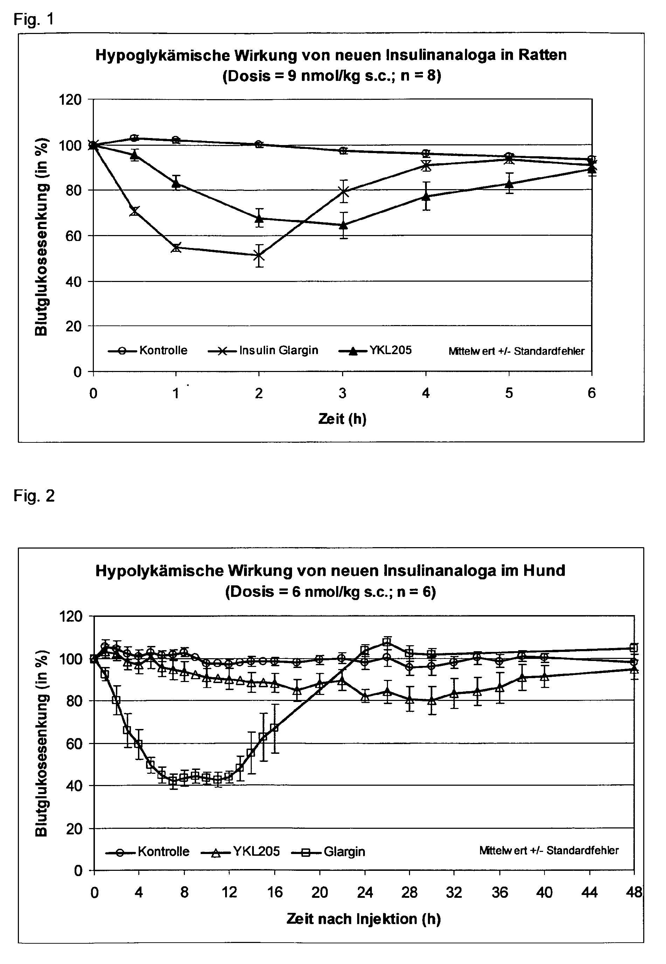

Während die ersten drei genannten Merkmale durch Einführen positiver Ladungen bzw. der Ausschaltung negativer Ladungen tendenziell Beiträge zu einer Erhöhung des pl - Wertes eines entsprechenden Insulinanalogs liefern, haben die letztgenannten Substitutionen und / oder Additionen negativ geladener Aminosäurereste den gegenteiligen Effekt und liefern Beiträge zu einer Erniedrigung des pl - Werts. Überraschenderweise haben gerade die oben beschriebenen Insulinanaloga die gewünschten vorteilhaften Zeit - / Wirkungsprofile. Die pl - Werte dieser Verbindungen sind niedriger als die der Verbindung 18 aus Kohn et al. (Arg (A0), Gly (A21), Arg (B31), Arg (B32) - Humaninsulin), weisen aber nichtsdestotrotz dabei einen verzögerten Wirkeintritt und eine längere Wirkdauer, d.h. einen extrem flachen und lang andauernden, gleichmäßigen Wirkungsverlauf auf. Damit wird die Gefahr von hypoglykämischen Ereignissen deutlich minimiert. Die Verzögerung ist so deutlich, dass sich der Effekt überraschend sogar in Modellversuchen an Ratten nachweisen lässt, obwohl im Gegensatz dazu die verzögerte Wirkung von Insulin Glargin in der Ratte nicht eindeutig beobachtbar ist. In Figur 1 ist die hypoglykämische Wirkung der erfindungsgemäßen Verbindung YKL205 verglichen mit der von Insulin Glargin dargestellt. Ähnliche Ergebnisse werden im Hund erhalten (siehe Figur 2). Damit sind neue Basalinsuline bereitgestellt worden, die deutlich weniger häufig appliziert werden müssen. Neben diesen beschriebenen pharmakokinetischen Vorteilen weisen die erfindungsgemäßen Analoga gegenüber Insulin Glargin deutlich bessere Eigenschaften in pharmakologischer Hinsicht wie z.B. Rezeptorspezifität und in vitro Mitogenität auf. Ferner zeigen die beanspruchten Insuline auch in physikalisch - chemischer Hinsicht Vorteile.

### Gegenstand der Erfindung ist somit ein Insulinanalogon der Formel I

wobei
- A0: Arg;
- A5: Asp, Gln oder Glu;
- A15: Asp, Glu oder Gln;
- A18: Asp, Glu oder Asn;
- B-1: Asp, Glu oder eine Aminogruppe;
- B0: Asp, Glu oder eine chemische Bindung;
- B1: Asp, Glu oder Phe;
- B2: Asp, Glu oder Val;
- B3: Asp, Glu oder Asn;
- B4: Asp, Glu oder Gln;
- B29: Lys;
- B30: Thr;
- B31: Arg oder Lys;
- B32: Arg-NH₂ oder Lys-NH₂
entspricht, wobei zwei Aminosäurereste der Gruppe enthaltend A5, A15, A18, B-1, B0, B1, B2, B3 und B4 gleichzeitig und unabhängig voneinander Asp oder Glu entsprechen.

Insbesondere sind Gegenstände der Erfindung Insulinanaloga wie oben ausgeführt, bei denen unabhängig voneinander A5 Glu entspricht, oder wobei A15 Glu entspricht, oder wobei A18 Asp entspricht, oder wobei B-1 einer Aminogruppe entspricht, oder wobei B0 Glu entspricht, oder wobei B1 Asp entspricht, oder wobei B2 Val entspricht, oder wobei B3 Asp entspricht, oder wobei B4 Glu entspricht.

Besonders bevorzugter Gegenstand der Erfindung ist ein Insulinanalogon ausgewählt aus einer Gruppe enthaltend:
Arg (A0), His (A8), Glu (A5), Asp (A18), Gly (A21), Arg (B31), Arg (B32) - NH₂ Humaninsulin,
Arg (A0), His (A8), Glu (A5), Asp (A18), Gly (A21), Arg (B31), Lys (B32) - NH₂ Humaninsulin,
Arg (A0), His (A8), Glu (A15), Asp (A18), Gly (A21), Arg (B31), Arg (B32) - NH₂ Humaninsulin,
Arg (A0), His (A8), Glu (A15), Asp (A18), Gly (A21), Arg (B31), Lys (B32) - NH₂ Humaninsulin,
Arg (A0), His (A8), Glu(A5), Glu (A15), Gly (A21), Arg (B31), Arg (B32) - NH₂ Humaninsulin,
Arg (A0), His (A8), Glu (A5), Glu (A15), Gly (A21), Arg (B31), Lys (B32) - NH₂ Humaninsulin,
Arg (A0), His(A8), Glu (A5), Gly (A21), Asp (B3), Arg (B31), Arg (B32) - NH₂ Humaninsulin,
Arg (A0), His(A8), Glu (A5), Gly (A21), Asp (B3), Arg (B31), Lys (B32) - NH₂ Humaninsulin,
Arg (A0), His (A8), Glu (A15), Gly (A21), Asp (B3), Arg (B31), Arg (B32) - NH₂ Humaninsulin,
Arg (A0), His (A8), Glu (A15), Gly (A21), Asp (B3), Arg (B31), Lys (B32) - NH₂ Humaninsulin,
Arg (A0), His (A8), Asp (A18), Gly (A21), Asp (B3), Arg (B31), Arg (B32) - NH₂ Humaninsulin,
Arg (A0), His (A8), Asp (A18), Gly (A21), Asp (B3), Arg (B31), Lys (B32) - NH₂ Humaninsulin,
Arg (A0), His(A8), Gly (A21), Asp (B3), Glu (B4), Arg (B31), Arg (B32) - NH₂ Humaninsulin,
Arg (A0), His (A8), Gly (A21), Asp (B3), Glu (B4), Arg (B31), Lys (B32) - NH₂ Humaninsulin,
Arg (A0), His (A8), Glu (A5), Gly (A21), Glu (B4), Arg (B31), Arg (B32) - NH₂ Humaninsulin,
Arg (A0), His (A8), Glu (A5), Gly (A21), Glu (B4), Arg (B31), Lys (B32) - NH₂ Humaninsulin,
Arg (A0), His (A8), Glu (A15), Gly (A21), Glu (B4), Arg (B31), Arg (B32) - NH₂ Humaninsulin,
Arg (A0), His (A8), Glu (A15), Gly (A21), Glu (B4), Arg (B31), Lys (B32) - NH₂ Humaninsulin,
Arg (A0), His (A8), Asp (A18), Gly (A21), Glu (B4), Arg (B31), Arg (B32) - NH₂ Humaninsulin,
Arg (A0), His (A8), Asp (A18), Gly (A21), Glu (B4), Arg (B31), Lys (B32) - NH₂ Humaninsulin,
Arg (A0), His (A8), Glu (A5), Gly (A21), Glu (B0), Arg (B31), Arg (B32) - NH₂ Humaninsulin,
Arg (A0), His (A8), Glu (A5), Gly (A21), Glu (B0), Arg (B31), Lys (B32) - NH₂ Humaninsulin,
Arg (A0), His (A8), Glu (A15), Gly (A21), Glu (B0), Arg (B31), Arg (B32) - NH₂ Humaninsulin,
Arg (A0), His (A8), Glu (A15), Gly (A21), Glu (B0), Arg (B31), Lys (B32) - NH₂ Humaninsulin,
Arg (A0), His (A8), Asp (A18), Gly (A21), Glu (B0), Arg (B31), Arg (B32) - NH₂ Humaninsulin,
Arg (A0), His (A8), Asp (A18), Gly (A21), Glu (B0), Arg (B31), Lys (B32) - NH₂ Humaninsulin,
Arg (A0), His (A8), Glu (A5), Gly (A21), Asp (B1), Arg (B31), Arg (B32) - NH₂ Humaninsulin,
Arg (A0), His (A8), Glu (A5), Gly (A21), Asp (B1), Arg (B31), Lys (B32) - NH₂ Humaninsulin,
Arg (A0), His (A8), Glu (A15), Gly (A21), Asp (B1), Arg (B31), Arg(B32) - NH₂ Humaninsulin,
Arg (A0), His (A8), Glu (A15), Gly (A21), Asp (B1), Arg (B31), Lys (B32) - NH₂ Humaninsulin,
Arg (A0), His (A8), Asp (A18), Gly (A21), Asp (B1), Arg (B31), Arg (B32) - NH₂ Humaninsulin,
Arg (A0), His (A8), Asp (A18), Gly (A21), Asp (B1), Arg (B31), Lys (B32) - NH₂ Humaninsulin,
Arg (A0), His (A8), Gly (A21), Glu (B0), Asp (B1), Arg (B31), Arg (B32) - NH₂ Humaninsulin,
Arg (A0), His (A8), Gly (A21), Glu (B0), Asp (B1), Arg (B31), Lys (B32) - NH₂ Humaninsulin,
Arg (A0), His (A8), Asp (A18), Gly (A21), Asp (B3), Arg (B30), Arg (B31) - NH₂ Humaninsulin,
Arg (A0), His (A8), Asp (A18), Gly (A21), Asp (B3), Arg (B30), Lys (B31) - NH₂ Humaninsulin.

Durch die Angabe des Begriffs "Humaninsulin" in den Bezeichnungen der genannten Insulinanaloga wird Bezug auf die Aminosäuresequenzen der A- und B-Kette von Humaninsulin genommen und alle Abweichungen (Additionen, Substitutionen, Deletionen) davon sind in einer gegebenen Bezeichnung eines Insulinanalogons angegeben.

Ein weiterer Gegenstand der Erfindung ist ein Verfahren zur Herstellung eines Insulinanalogons wie oben genannt, wobei ein Vorläufer des Insulinanalogs rekombinant hergestellt wird, der Vorläufer enzymatisch zu zwei-kettigem Insulin prozessiert wird und eine Kupplung mit Argininamid in Gegenwart eines Enzyms mit Trypsinaktivität durchgeführt wird, und das Insulinanalogon isoliert wird.

Ein weiterer Gegenstand der Erfindung ist eine Verwendung eines Insulinanalogons wie oben beschrieben zur Herstellung eines Medikaments zur Behandlung von Diabetes, insbesondere von Diabetes Typ I oder Typ II. Ebenfalls ist Gegenstand der Erfindung eine Verwendung eines Insulinanalogons wie oben beschrieben zur Herstellung eines Medikaments zur Unterstützung der beta -Zellregeneration

Ein weiterer Gegenstand der Erfindung ist ein Arzneimittel enthaltend ein Insulinanalogon wie oben beschrieben und/oder physiologisch verträgliche Salze davon.

Ein weiterer Gegenstand der Erfindung ist eine Formulierung des Insulinanalogons wie oben beschrieben, wobei die Formulierung in wässriger Form enthaltend das gelöste Insulinanalog vorliegt.

Ein weiterer Gegenstand der Erfindung ist eine Formulierung des Insulinanalogons wie oben beschrieben, wobei die Formulierung in Form von Pulver vorliegt.

Ein weiterer Gegenstand der Erfindung ist eine Formulierung wie oben beschrieben, wobei das Insulinanalogon wie oben beschrieben in kristalliner und/oder amorpher Form vorhanden ist.

Ein weiterer Gegenstand der Erfindung ist eine Formulierung des Insulinanalogons wie oben beschrieben, wobei die Formulierung in Form einer Suspension vorliegt. Ein weiterer Gegenstand der Erfindung ist eine Formulierung des Insulinanalogons wie oben beschrieben, wobei die Formulierung zusätzlich ein chemisches Chaperon enthält.

Ein weiterer Gegenstand der Erfindung ist eine DNA kodierend für die A-Kette oder B-Kette eines Insulinanalogons wie oben beschrieben.

Ein weiterer Gegenstand der Erfindung ist ein Vektor enthaltend eine DNA wie oben beschrieben.

Ein weiterer Gegenstand der Erfindung ist ein Wirtsorganismus enthaltend eine DNA wie oben beschrieben oder einen Vektor wie oben beschrieben.

Ein weiterer Gegenstand der Erfindung ist eine Formulierung wie oben beschrieben, bei der noch zusätzlich ein Glucagon-Like Peptide-1 (GLP1) oder ein Analogon oder Derivat davon, oder Exendin-3 bzw. -4 oder ein Analogon oder Derivat davon, vorzugsweise Exendin-4 enthalten ist.

Ein weiterer Gegenstand der Erfindung ist eine Formulierung wie oben beschrieben, bei dem ein Analogon von Exendin-4 ausgewählt wird aus einer Gruppe enthaltend
H-desPro³⁶-Exendin-4-Lys₆-NH₂,
H-des(Pro^{36,37})-Exendin-4-Lys₄-NH₂ und
H-des(Pro^{36,37})-Exendin-4-Lys₅-NH₂,
oder ein pharmakologisch tolerierbares Salz davon.

Ein weiterer Gegenstand der Erfindung ist eine Formulierung wie oben beschrieben, bei dem ein Analogon von Exendin-4 ausgewählt wird aus einer Gruppe enthaltend
desPro³⁶ [Asp²⁸]Exendin-4 (1-39),
desPro³⁶ [IsoAsp²⁸]Exendin-4 (1-39),
desPro³⁶ [Met(O)¹⁴, Asp²⁸]Exendin-4 (1-39),
desPro³⁶ [Met(O)¹⁴, IsoAsp²⁸]Exendin-4 (1-39),
desPro³⁶ [Trp(O₂)²⁵, Asp²⁸]Exendin-2 (1-39),
desPro³⁶ [Trp(O₂)²⁵, IsoAsp²⁸]Exendin-2 (1-39),
desPro³⁶ [Met(O)¹⁴Trp(O₂)²⁵, Asp²⁸]Exendin-4 (1-39) und
desPro³⁶ [Met(O)¹⁴Trp(O₂)²⁵, IsoAsp²⁸]Exendin-4 (1-39),
oder ein pharmakologisch tolerierbares Salz davon.

Ein weiterer Gegenstand der Erfindung ist eine Formulierung wie im vorigen Absatz beschrieben, bei denen an die C-Termini der Analoga von Exendin-4 das Peptid-Lys₆-NH₂ angefügt ist.

Ein weiterer Gegenstand der Erfindung ist eine Formulierung wie oben beschrieben, bei dem ein Analogon von Exendin-4 ausgewählt wird aus einer Gruppe enthaltend
H-(Lys)₆- des Pro³⁶ [Asp²⁸]Exendin-4(1-39)-Lys₆-NH₂
des Asp²⁸Pro³⁶, Pro³⁷, Pro₃₈ Exendin-4(1-39) -NH₂,
H-(Lys)₆- des Pro³⁶, Pro³⁷, Pro³⁸ [Asp²⁸]Exendin-4(1-39) -NH₂,
H-Asn-(Glu)₅ des Pro³⁶, Pro³⁷, Pro³⁸ [Asp²⁸]Exendin-4(1-39) -NH₂,
des Pro³⁶, Pro³⁷, Pro³⁸ [Asp²⁸]Exendin-4(1-39)-(Lys)₆-NH₂,
H-(Lys)₆- des Pro³⁶, Pro³⁷, Pro³⁸ [Asp²⁸]Exendin-4(1-39)-(Lys)₆-NH₂,
H-Asn-(Glu)₅- des Pro³⁶, Pro³⁷, Pro³⁸ [Asp²⁸]Exendin-4(1-39)-(Lys)₆-NH₂,
H-(Lys)₆- des Pro³⁶ [Trp(O₂)²⁵, Asp²⁸]Exendin-4(1-39)-Lys₆-NH₂,
H- des Asp²⁸ Pro³⁶, Pro³⁷, Pro³⁸ [Trp(O₂)²⁵]Exendin-4(1-39) -NH₂,
H-(Lys)₆- des Pro³⁶, Pro³⁷, Pro³⁸ [Trp(O₂)²⁵, Asp²⁸]Exendin-4(1-39) -NH₂,
H-Asn-(Glu)₅- des Pro³⁶, Pro³⁷, Pro³⁸ [Trp(O₂)²⁵, Asp²⁸]Exendin-4(1-39) -NH₂,
des Pro³⁶, Pro³⁷, Pro³⁸ [Trp(O₂)²⁵, Asp²⁸]Exendin-4(1-39)-(Lys)₆-NH₂,
H-(Lys)₆-des Pro³⁶, Pro³⁷, Pro³⁸ [Trp(O₂)²⁵, Asp²⁸]Exendin-4(1-39)-(Lys)₆-NH₂,
H-Asn-(Glu)₅- des Pro³⁶, Pro³⁷, Pro³⁸ [Trp(O₂)²⁵, Asp²⁸]Exendin-4(1-39)-(Lys)₆-NH₂,
H-(Lys)₆- des Pro³⁶ [Met(O)¹⁴, Asp²⁸]Exendin-4(1-39)-Lys₆-NH₂,
des Met(O)¹⁴ Asp²⁸ Pro³⁶, Pro³⁷, Pro³⁸ Exendin-4(1-39) -NH₂,
H-(Lys)₆- des Pro³⁶, Pro ³⁷, Pro³⁸ [Met(O)¹⁴, Asp²⁸]Exendin-4(1-39) -NH₂,
H-Asn-(Glu)₅- des Pro³⁶, Pro³⁷, Pro³⁸ [Met(O)¹⁴, Asp²⁸] Exendin-4(1-39) -NH₂,
des Pro³⁶, Pro³⁷, Pro³⁸ [Met(O)¹⁴, Asp²⁸]Exendin-4(1-39)-(Lys)₆-NH₂,
H-(Lys)₆- des Pro³⁶, Pro³⁷, Pro³⁸ [Met(O)¹⁴, Asp²⁸]Exendin-4(1-39)-Lys₆-NH₂,
H-Asn-(Glu)₅ des Pro³⁶, Pro³⁷, Pro³⁸ [Met(O)¹⁴, Asp²⁸] Exendin-4(1-39)-(Lys)₆-NH₂,
H-(Lys)₆- des Pro³⁶ [Met(O)¹⁴, Trp(O₂)²⁵, Asp²⁸]Exendin-4(1-39)-Lys₆-NH₂,
des Asp²⁸ Pro³⁶, Pro³⁷, Pro³⁸ [Met(O)¹⁴, Trp(O₂)²⁵]Exendin-4(1-39) -NH₂,
H-(Lys)₆- des Pro³⁶, Pro³⁷, Pro³⁸ [Met(O)¹⁴, Trp(O₂)²⁵, Asp²⁸]Exendin-4(1-39) -NH₂,
H-Asn-(Glu)₅- des Pro³⁶, Pro³⁷, Pro³⁸ [Met(O)¹⁴, Asp²⁸] Exendin-4(1-39) -NH₂,
des Pro³⁶, Pro³⁷, Pro³⁸ [Met(O)¹⁴, Trp(O₂)²⁵, Asp²⁸]Exendin-4(1-39)-(Lys)₆-NH₂,
H-(Lys)₆- des Pro³⁶, Pro³⁷, Pro³⁸ [Met(O)¹⁴, Trp(O₂)²⁵, Asp²⁸]Exendin-4(1-39)-(Lys)₆-NH₂,
H-Asn-(Glu)₅- des Pro³⁶, Pro³⁷, Pro³⁸ [Met(O)¹⁴, Trp(O₂)²⁵, Asp²⁸] Exendin-4(1-39)-(Lys)₆-NH₂, oder ein pharmakologisch tolerierbares Salz davon.

Ein weiterer Gegenstand der Erfindung ist eine Formulierung wie oben beschrieben, bei dem zusätzlich Arg³⁴, Lys²⁶ (N^{ε}(γ-glutamyl(N^{α}-hexadecanoyl))) GLP-1 (7-37) [liraglutide] oder ein pharmakologisch tolerierbares Salz davon enthalten ist.

Dem Fachmann ist dabei klar, dass die erfindungsgemäßen Insuline Gegenstand einer pharmazeutischen Formulierung sein können, die nach Applikation vorteilhaft wirkt. Dabei geht man von wässrigen Lösungen aus. Entsprechend müssen weitere Komponenten mischbar sein. Die Gefahr viraler tierischer Kontamination wird dadurch minimiert, dass die Zubereitung keine Komponenten enthalten sollte, die aus tierischen Quellen stammen. Es ist weiterhin vorteilhaft, durch Zusatz von Konservierungsmitteln eine mikrobielle Verunreinigung zu verhindern. Durch den Zusatz isotoner Agentien kann eine mögliche negative Auswirkung der Formulierung auf die Physiologie der Gewebezellen an der Applikationsstelle kompensiert werden. Stabilisierend kann sich der Zusatz von Protamin auswirken, so dass man weitgehend salzfreien Insulinzubereitung gelangen kann, wenn man der Formulierung Protamin zufügt. Der Zusatz von einer phenolischen Komponente kann zu einer Stabilisierung der Struktur des verwendeten Insulinanalogons führen und so unter anderem den Verzögerungseffekt beim Wirkungseintritt zusätzlich bewirken. Der Formulierung zugesetzt können auch Substanzen sein, die die Raumstruktur der erfindungsgemäßen Verzögerungsinsuline stabilisieren und zu besserer thermischen Stabilität führen. Solche chemischen Chaperone können z.B. kurze synthetische Peptide, die auch Aminosäureanaloga enthalten können oder z.B. vom C-Peptid des Insulin abgeleitete Peptidsequenzen umfassen.

Zur Entwicklung von Depotformen können die erfindungsgemäßen Insuline in Nanopartikel eingebunden werden. Denkbar sind auch sogenannte "Slow release" Formuierungen, bei denen das erfindungsgemäße Verzögerungsinsulin reversibel an polymere Träger gebunden vorliegt.

Die erfindungsgemäßen Insuline können parallel zu schnellwirksamen Insulinen wie Apidra^{®}, NovoRapid^{®}, Humalog^{®} oder sich in Entwicklung befindlichen Insulinderivaten oder Formulierungen mit entsprechendem Zeit - / Aktionsprofil oder inhalierbarem Insulin oder nasal oder oral applizierten Insulinen, die sich in Entwicklung befinden, verabreicht werden. Dabei ist es dem Fachmann klar, dass dazu auch entsprechend formulierte Mischungen aus schnellwirksamen und erfindungsgemäßem Verzögerungsinsulin verwendet werden können. Weiterhin können die erfindungsgemäßen Insuinanaloga in pharmazeutischen Zubereitungen verwendet werden, die Peptide, die durch eine dem GLP-1 (Glucagon like Peptide-1) oder dem Exendin-4 bzw. Exendin-3 vergleichbare Aktivität beschrieben sind, enthalten. Beispiele für solche Peptide stellen GLP-1 (7-37), Exenatide (Byetta^{®}) oder Peptide, deren Herstellung in den Patentanmeldungen WO 2006/058620, WO 2001/04156, WO 2004/005342 und WO 98/08871 beschrieben ist, dar. Besonders vorteilhaft sind dabei Formulierungen, die eine Depotformulierung dieser Peptide enthalten. Vorteilhaft besonders in der Anfangsphase der Typll Diabetes Erkrankung sind Therapieformen, die parallel zu Verabreichung der erfindungsgemäßen Pharmazeutika vorsehen, die die Insulinwirkung erhöhen, wie z.B. Metformin. Kombinationstherapien mit Dipeptidyl Peptidase-4 Inhibitoren, die den Spiegel an Inkretinen erhöhen, sind wie Kombinationen mit Sulfonylharnstoffen, die die Insulinausschüttung im Pankreas erhöhen, ebenfalls möglich. Besonders vorteilhaft können die erfindungsgemäßen Verzögerungsinsuline dann eingesetzt werden, wenn durch Applikation von Differenzierungsfaktoren, die Regeneration von pankreatischen Betazellen aus entsprechenden Stammzellen eingeleitet wird. All diese Anwendungen sind beispielhaft für die Therapie des Diabetes genannt und ebenfalls Gegenstand der Erfindung. Ein weiterer Gegenstand der Erfindung ist somit die Verwendung der erfindungsgemäßen Insuline in Kombination mit anderen Wirkstoffen zur Behandlung von Diabetes, insbesondere Diabetes Typ I oder Typ II Diabetes.

Ein weiterer Gegenstand der Erfindung ist ein Arzneimittel, das ein erfindungsgemäßes Insulinanalogon enthält, welches insbesondere eine wässrige Formulierung oder ein Pulver darstellt.

Das Arzneimittel ist eine pharmazeutische Zubereitung, die vorzugsweise eine Lösung oder Suspension zu Injektionszwecken ist; sie ist gekennzeichnet durch einen Gehalt an mindestens einem erfindungsgemäßen Insulinanalog, und/oder mindestens einem von deren physiologisch verträglichen Salzen in gelöster, amorpher und/oder kristalliner - vorzugsweise in gelöster -Form.

Die Zubereitung weist vorzugsweise einen pH-Wert zwischen etwa 2,5 und 8,5, insbesondere zwischen etwa 4,0 und 8,5 auf, enthält vorzugsweise ein geeignetes Isotonisierungsmittel, ein geeignetes Konservierungsmittel und gegebenenfalls einen geeigneten Puffer, sowie vorzugsweise auch eine bestimmte Zinkionen-Konzentration, in steriler wässriger Lösung. Die Gesamtheit der Zubereitungsbestandteile außer dem Wirkstoff bildet den Zubereitungs-Träger. Geeignete Isotonisierungsmittel sind z.B. Glycerin, Glukose, Mannit, NaCl, Calcium-oder Magnesium-Verbindungen wie CaCl₂ etc. Durch die Wahl des Isotonisierungsmittels und/oder Konservierungsstoffes beeinflusst man die Löslichkeit der erfindungsgemäßen Insuline bzw. deren physiologisch verträgliche Salze bei schwach sauren pH-Werten.
Geeignete Konservierungsmittel sind z.B. Phenol, m-Cresol, Benzylalkohol und/oder p-Hydroxybenzoesäureester.

Als Puffersubstanzen, insbesondere zur Einstellung eines pH-Wertes zwischen etwa 4,0 und 8,5 können z.B. Natriumacetat, Natriumcitrat, Natriumphosphat etc. verwendet werden. Ansonsten sind zur Einstellung des pH-Wertes auch physiologisch unbedenkliche verdünnte Säuren (typischerweise HCl) bzw. Laugen (typischerweise NaOH) geeignet.

Wenn die Zubereitung einen Zinkgehalt besitzt, ist ein solcher von 1 bis 2 mg/ml, insbesondere von 1 µg/ml bis 200 µg Zink/ml bevorzugt.
Über den Zusatz von Zn lässt sich das Wirkprofil der erfindungsgemäßen Insulinanaloge überraschend gut beeinflussen. Dies erlaubt Zubereitungen, die sich in Bezug auf Gesamtwirkungsdauer, die Geschwindigkeit des Wirkungseintritts und das Profil der Wirkungskurve unterscheiden und so eine individuelle Einstellung des Patienten erlauben. Eine andere Möglichkeit ergibt sich durch den Einsatz eines "Zweikammer - Insulin - Device", das die Applikation einer Formulierung mit schnellem Wirkeintritt und / oder langsamem flachen Wirkeintritt je nach Lebenssituation erlaubt.

Zwecks Variation des Wirkstoffprofils der erfindungsgemäßen Zubereitung kann auch unmodifiziertes Insulin, vorzugsweise Rinder-, Schweine- oder Human-Insulin, insbesondere Humaninsulin, oder Insulinanaloga und Derivate davon zugemischt werden. Ebenfalls können ein oder mehrere Exendin-4 Derivate oder Peptide, die durch eine dem GLP-1 (Glucagon like peptide-1) vergleichbare Aktivität charakterisiert sind, oder direkt GLP-1 entsprechen, zugemischt werden. Solche Arzneimittel (Zubereitungen) sind ebenfalls Gegenstand der Erfindung.

Bevorzugte Wirkstoffkonzentrationen sind solche entsprechend etwa 1 - 1500, weiter bevorzugt etwa 5-1000 und insbesondere etwa 40 - 400 internationale Einheiten/ml.

Die erfindungsgemäßen Insulinanaloga werden zunächst als Vorstufe, die noch nicht das Amid umfasst, biotechnologisch hergestellt. Dem Fachmann ist geläufig, das es eine Vielzahl von Möglichkeiten zur Herstellung von Insulinen gibt. Als Wirtszellsysteme finden dabei Bakterien, Hefen und Pflanzen bzw. fermentativ zu kultivierende Pflanzenzellen Verwendung. Falls die Kostenbetrachtung es erlaubt sind auch Expressionssysteme, die tierische Zellen als Wirtssystem nutzen, denkbar. Voraussetzung dafür ist aber eine sichere Freiheit von tierischen Viren. Somit ist klar, dass die beispielhaft beschriebenen Expressionssysteme nur einen kleinen Ausschnitt der für die rekombinante Herstellung von Proteinen entwickelten Wirts/Vektorsysteme darstellen. In der Anmeldung werden z.B. biotechnologische Verfahren, die Hefe- oder Pflanzensysteme wie Moose, Algen oder höhere Pflanzen wie Tabak, Erbse, Distel, Gerste, Mais oder Raps zur Grundlage haben nicht beschrieben. Dennoch sind Wirts/Vektor Systeme sowie kodierende DNA - Sequenzen, die die Herstellung der Zielpeptide in entsprechenden biotechnologischen Expressionssystemen erlauben, ebenfalls Bestandteil der Erfindung. Wirtsorganismen können also insbesondere ausgewählt werden aus dem Pflanzenreich aus Organismen der ersten Abteilung Schizophyta enthaltend Schizomycetes, Bakterien oder Blaualgen, Organismen der 2. Abteilung Phycophyta V. Klasse Chlorophyceae, Organismen der 2. Abteilung Phycophyta VII. Klasse Rhodophyceae, Organismen der 3. Abteilung Mycophyta, Organismen der 5. Abteilung Bryophyta und Organismen der 7. Abteilung Spermatophyta.

In der Europäischen Patentanmeldung EP-A 1 222 207 ist ein Plasmid pINT358d beschrieben, das für ein Prä - Proinsulin codiert, welches ein verändertes C-Peptid umfasst. Mit Hilfe der Polymerase Kettenreaktion (PCR) ist es nun möglich, gezielt die das Proinsulin kodierende Sequenz so zu verändern, dass Prä - Proinsuline exprimiert werden können, die als Vorstufen für die erfindungsgemäßen Insuline dienen können. Entsprechende Fusionsproteine müssen nicht notwendigerweise intrazellulär hergestellt werden. Es ist dem Fachmann klar, dass solche Proteine auch durch bakterielle Expression mit anschließender Sekretion in das Periplasma und/oder in den Kulturüberstand hergestellt werden können. Die Europäische Patentanmeldung EP-A 1 364 029 beschreibt dies beispielhaft. Die Proinsulinvorstufen, die zu den erfindungsgemäßen Analoga führen, sind ebenfalls Gegenstand der Erfindung.

Die so hergestellten Proinsuline können prinzipiell zu einer Insulinanalogavorstufe umgewandelt werden, die in Position A0 Lysin oder Arginin umfasst und am C-terminalen Ende der B - Kette Lysin oder Arginin trägt.

Liegen die erfindungsgemäßen Proinsuline nach intrazellulärer Expression in Bakterien als Einschlusskörper oder löslich vor, müssen diese Vorstufen durch in vitro Faltung in die richtige Konformation gefaltet werden, bevor die Prozessierung und biochemische Modifikation vorgenommen werden kann. Dabei erlaubt das beschriebene Fusionsprotein eine direkte Faltung nach Denaturierung mittels Harnstoff oder Guanidinium Hydrochlorid, Faltungsintermediate sind dabei ebenfalls Gegenstand der Erfindung.

Zur Anreicherung der einzelnen Zwischenstufen finden biochemische Methoden insbesondere Trennverfahren Verwendung, deren zugrunde liegenden Prinzipien publiziert und sogar Gegenstand von Lehrbüchern sind. Dem Fachmann ist klar, dass solche Prinzipien in Folge kombiniert werden können und so zu Verfahren führen können, die in ihre Abfolge vorher nicht publiziert wurden. Verfahren, die zur Reinigung der erfindungsgemäßen Analoga führen sind somit ebenfalls Gegenstand der Erfindung.

Ein weiterer Gegenstand der Erfindung ist ein Verfahren zur Herstellung der erfindungsgemäßen Insulinanaloga, wobei ein Vorläufer des Insulinanalogons rekombinant hergestellt und enzymatisch zu einer 2 - kettigen Insulinvorstufe umgewandelt wird, die N-terminal zu Aminosäure 1 der A- Kette Arginin bzw. Lysin trägt und am C-terminalen Ende der B-Kette einen Lysin oder Argininrest aufweist, der mit Argininamid oder Lysinamid in Gegenwart eines Enzyms mit Trypsinaktivität in das Amid und somit in das erfindungsgemäße Verzögerungsinsulin überführt und über ein biochemischer Reinigungsverfahren hochrein dargestellt wird.

Proteine, welche sich durch Substitution wenigstens eines natürlich auftretenden Aminosäurerestes mit anderen Aminosäureresten und/oder Addition und/oder Entfernen wenigstens eines Aminosäurerestes von dem entsprechenden, ansonsten gleichen natürlich vorkommenden Protein unterscheiden, werden als "Analoga" von Proteinen bezeichnet. Dabei kann es sich bei den hinzugefügten und / oder ersetzten Aminosäureresten auch um solche handeln, die nicht natürlich vorkommen.

Proteine, welche durch chemische Modifizierung bestimmter Aminosäurereste von Ausgangsproteinen erhalten werden, bezeichnet man als "Derivate" von Proteinen. Die chemische Modifikation kann z.B. in der Addition einer oder mehrerer bestimmter chemischer Gruppen an eine oder mehrere Aminosäuren bestehen.

Figurenlegende:
- Fig. 1:: Blutzuckersenkende Wirkung von neuen Insulinanaloga in Ratten
- Fig. 2:: Blutzuckersenkende Wirkung von neuen Insulinanaloga im Hund
- Fig. 3:: Blutzuckersenkende Wirkung von YKL205 im Hund
- Fig. 4:: Zinkabhängigkeit der hypoglykämischen Wirkung von YKL205 im Hund

Die folgenden Beispiele sollen den Erfindungsgedanken illustrieren, ohne dabei beschränkend zu wirken.

### Beispiel 1: Herstellung des Vektorderivates pINT3580, das für Gly (A21) - Insulin und ein modifiziertes C-Peptid, das an der C/A- Kettengrenze Arg Arg trägt, kodiert.

Die Europäische Patentanmeldung EP-A 1 222 207 beschreibt die Plasmide pINT358d, pINT91d und die Primer- Sequenz Tir. DNA dieser Produkte findet Verwendung in der Konstruktion des Plasmides pINT3580. Das Plasmid pINT358d ist dabei durch eine Gensequenz charakterisiert, die für ein modifiziertes C-Peptid mit besonderen Eigenschaften kodiert. Drei Primersequenzen werden synthetisiert:
pint3580_glya21rev
5'- CAAAGGTCGACTATTA**GCC**GCAGTAGTTCTCCAGCTGG-3' (SEQ ID NO: 3)

Dieser Primer dient nach Aufarbeitung dazu in Position 21 der A-Kette der von pINT358d kodierten Proinsulinsequenz Glycin (fett gedruckt, unterstrichen) anstelle von Asparagin einzuführen.
arg_cjuncf
5'-GTCCCTGCAG**CGT**CGCGGCATCGTGGAGCAG-3' (SEQ ID NO: 4)

Dieser Primer dient, wie der Primer arg_cjunc_rev, zur Einführung von Arginin anstelle von Lysin an der Insulin A-/ B- Kettengrenze.
arg_cjunc_rev
5'- CCACGATGCC GCGACGCTGC AGGGACCCCT CCAGCG-3' (SEQ ID NO: 5)

Das Codon für das einzuführende Arginin ist in beiden Primern fett gedruckt.
Mit DNA des Plasmides pINT358d als Matrize wird mit den Primerpaaren Tir / arg_cjunc_rev und arg_cjuncf / pint3580_glya21rev entsprechend der Europäischen Patentanmeldung EP-A 1 222 207 je eine PCR durchgeführt. Aliquots der Produkte beider Reaktionen werden kombiniert und zusammen mit dem Primerpaar Tir / pint3580_glya21rev in einer dritten PCR eingesetzt. Das Produkt dieser Reaktion wird nach gelektrophoretischer Auftrennung des Reaktionsgemisches gereinigt und mit den Restriktionsenzymen Sal1 / Nco1 nach Angaben des Herstellers in ein und derselben Reaktion verdaut, das Reaktionsgemisch gelelektrophoretisch aufgetrennt und das die Proinsulinsequenz kodierende DNA - Fragment isoliert. Das Fragment wird anschließend über eine DNA- Ligasereaktion in die Nco1 / Sal1 geöffnete pINT91d Vektor DNA insertiert.

Mit dem Ligationsgemisch werden kompetente E. coli Bakterienzellen transformiert. Das Transformationsgemisch wird auf Selektionsplatten, die 25 mg/ I Ampicillin enthalten, ausplattiert. Plasmid DNA wird von Kolonien isoliert und mittels DNA-Sequenzanalyse charakterisiert. Richtige Plasmide erhalten die Bezeichnung pINT3580.

### Beispiel 2: Konstruktion des Plasmides pINT3581 kodierend für His (A8), Gly (A21) - Präproinsulin

Die Konstruktion erfolgt wie in Beispiel 1 beschrieben über 3 Polymerase Kettenreaktionen. Das Produkt der dritten Reaktion wird nach Nco1/ Sal1 Spaltung in die Nco1 / Sal1 geöffnete pINT91d Vektor DNA insertiert. Verwendet werden die Primer Tir und pint3580_glya21rev. Zwei weitere Primer werden synthetisiert:
pint3580_Ha8f
5'-AGCAGTGCTGC**CAC**AGCATCTGCTCCCTCTAC-3' (SEQ ID NO: 6)
pint3580_Ha8rev
5'-GAG CAGATGCT GTG GCAGCACTG CTCCACGATG-3' (SEQ ID NO: 7)

Das Codon, das für Histidin in Position 8 der A- Kette kodiert ist jeweils fett herausgestellt. Die Konstruktion wird wie ins Beispiel 1 beschrieben durchgeführt. Template für PCR1 und 2 ist DNA des Plasmides pINT3580. PCR1 wird mit dem Primerpaar Tir/ pint3580_Ha8rev und PCR2 mit dem Primerpaar pint3580_Ha8f/ pint3580_glya21rev durchgeführt. In PCR3 wird das Primerpaar Tir/ pint3580_glya21rev eingesetzt. Template ist dabei ein Gemisch der Reaktionsprodukte von PCR1 und PCR2. Richtige Plasmide erhalten die Bezeichnung pINT3581.

### Beispiel 3: Konstruktion des Plasmides pINT3582 kodierend für His (A8), Glu (A5), Gly (A21) - Präproinsulin

Die Konstruktion erfolgt wie in Beispiel 1 und 2 beschrieben über 3 Polymerase Kettenreaktionen. Das Produkt der dritten Reaktion wird nach Nco1/ Sal1 Spaltung in die Nco1 / Sal1 geöffnete pINT91d Vektor DNA insertiert. Verwendet werden die Primer Tir und pint3580_glya21rev. Zwei weitere Primer werden synthetisiert:
pint3581_Ea5f
5'GCATCGTGGAG**GAG**TGCTGCCACAGCATCTG 3' (SEQ ID NO: 8) pint3581_Ea5rev
5'-CTGT GGCAGCACTC CTCCACGATG CCGCGACG-3' (SEQ ID NO: 9)

Das Codon, das für Glutaminsäure in Position 5 der A- Kette kodiert, ist jeweils fett herausgestellt. Die Konstruktion wird wie ins Beispiel 1 beschrieben durchgeführt. Template ist DNA des Plasmides pINT3581. Richtige Plasmide erhalten die Bezeichnung pINT3582.

### Beispiel 4: Konstruktion des Plasmides pINT3583 kodierend für His (A8), Asp (A18), Gly(A21) - Präproinsulin

Die Konstruktion erfolgt abweichend von Beispiel 1 über nur eine Polymerase Kettenreaktion. Das Produkt dieser Reaktion wird nach Nco1/ Sal1 Spaltung in die Nco1 / Sal1 geöffnete pINT91d Vektor DNA insertiert. Verwendet wird der Primer Tir. Ein weiterer Primer wird synthetisiert:
pint3580_Da18rev
5' CAAAGGTCGACTATTAGCCGCAGTA**GTC**CTCCAGCTGGTAGAGGGAG 3' (SEQ ID NO: 10)

Das Codon, das für Asparaginsäure in Position 18 der A- Kette kodiert, ist fett herausgestellt. Template ist DNA des Plasmides pINT3581. Richtige Plasmide erhalten die Bezeichnung pINT3583.

### Beispiel 5: Konstruktion des Plasmides pINT3584 kodierend für His (A8), Glu (A5) Asp (A18), Gly (A21) - Präproinsulin

Die Konstruktion erfolgt abweichend von Beispiel 1 über nur eine Polymerase Kettenreaktion. Das Produkt dieser Reaktion wird nach Nco1/ Sal1 Spaltung in die Nco1 / Sal1 geöffnete pINT91d Vektor DNA insertiert. Verwendet wird der Primer Tir. pint3580_Da18rev (Bsp.4). Template ist DNA des Plasmides pINT3582. Richtige Plasmide erhalten die Bezeichnung pINT3584. Das von dem Plasmid kodierte Prä-Proinsulin ist Vorstufe für die Verbindung YKL205-1, die nach Amidierung mit Argininamid entsteht und die folgende Struktur beschreibt:
Arg (A0), Glu (A5), His (A8), Asp(A18), Gly(A21), Arg(B31), Arg(B32)-NH₂-Humaminsulin

Entsprechende Amidierung mit Lysinamid führt zur Verbindung YKL205-1b:
Arg (A0), Glu (A5), His (A8), Asp (A18), Gly(A21), Arg (B31), Lys (B32)-NH₂-Humaninsulin

### Beispiel 6: Konstruktion des Plasmides pINT3585 kodierend für His (A8), Glu (A15), Gly (A21) - Präproinsulin

Die Konstruktion erfolgt abweichend von Beispiel 1 über nur eine Polymerase Kettenreaktion. Das Produkt dieser Reaktion wird nach Nco1/ Sal1 Spaltung in die Nco1 / Sal1 geöffnete pINT91d Vektor DNA insertiert. Verwendet wird der Primer Tir. Ein weiterer Primer wird synthetisiert:
pint3580_Ea15rev

Das Codon, das für Glutaminsäure in Position 15 der A- Kette kodiert, ist fett herausgestellt. Template ist DNA des Plasmides pINT3581. Richtige Plasmide erhalten die Bezeichnung pINT3585.

### Beispiel 7: Konstruktion des Plasmides pINT3586 kodierend für His (A8), Glu (A15), Asp (A18), Gly (A21) - Präproinsulin

Die Konstruktion erfolgt abweichend von Beispiel 1 über nur eine Polymerase Kettenreaktion. Das Produkt dieser Reaktion wird nach Nco1/ Sal1 Spaltung in die Nco1 / Sal1 geöffnete pINT91d Vektor DNA insertiert. Verwendet wird der Primer Tir. Ein weiterer Primer wird synthetisiert:
pint3585_Ea15_Da18rev

Das Codon, für Glutaminsäure in Position 15 der A- Kette und Asparaginsäure in Position A18 der A- Kette ist jeweils fett herausgestellt. Template ist DNA des Plasmides pINT3581. Richtige Plasmide erhalten die Bezeichnung pINT3586. Das von dem Plasmid kodierte Prä - Proinsulin ist Vorstufe für die Verbindung YKL205, die nach Amidierung mit Argininamid entsteht und die folgende Struktur beschreibt:
Arg (A0), His (A8), Glu (A15), Asp (A18), Gly (A21), Arg (B31), Arg (B32) - NH₂-Humaninsulin

Das von dem Plasmid kodierte Prä - Proinsulin ist Vorstufe für die Verbindung YKL205b, die nach Amidierung mit Lysinamid entsteht und die folgende Struktur beschreibt:
Arg (A0), His (A8), Glu (A15), Asp (A18), Gly (A21), Arg (B31), Lys (B32) - NH₂-Humaninsulin

### Beispiel 8: Konstruktion des Plasmides pINT3587kodierend für Glu (A5), His (A8), Glu (A15), Gly (A21) - Präproinsulin

Die Konstruktion erfolgt abweichend von Beispiel 1 über nur eine Polymerase Kettenreaktion. Das Produkt dieser Reaktion wird nach Nco1/ Sal1 Spaltung in die Nco1 / Sal1 geöffnete pINT91d Vektor DNA insertiert. Verwendet wird der Primer Tir und pint3580_Ea15rev gemäß Beispiel 6. Template ist DNA des Plasmides pINT3582. Richtige Plasmide erhalten die Bezeichnung pINT3587. Das von dem Plasmid kodierte Prä - Proinsulin ist Vorstufe für die Verbindung YKL205 -2, die nach Amidierung mit Argininamid entsteht und die folgende Struktur beschreibt:
Arg (A0), Glu (A5), His (A8), Glu (A15), Gly (A21), Arg (B31), Arg (B32) - NH₂-Humaninsulin

Das von dem Plasmid kodierte Prä - Proinsulin ist Vorstufe für die Verbindung YKL205 -2b, die nach Amidierung mit Lysinamid entsteht und die folgende Struktur beschreibt:
Arg (A0), Glu (A5), His (A8), Glu (A15), Gly (A21), Arg (B31), Lys (B32) - NH₂-Humaninsulin

### Beispiel 9: Konstruktion des Plasmides pINT3588 kodierend für His (A8), Gly (A21), Asp (B3)- Präproinsulin

Die Konstruktion erfolgt wie in Beispiel 1 und 2 beschrieben über 3 Polymerase Kettenreaktionen. Das Produkt der dritten Reaktion wird nach Nco1/ Sal1 Spaltung in die Nco1 / Sal1 geöffnete pINT91d Vektor DNA insertiert. Verwendet werden die Primer Tir und pint3580_glya21rev. Zwei weitere Primer werden synthetisiert:
pint3581_Db3f
5'- GCACGATTTGTG**GAC**CAGCACCTGTGCGGC -3' (SEQ ID NO: 13)
pint3581_Db3rev
5'- CACAGG TGCTG**GTC**CA CAAATCGTGC CGAATTTC -3' (SEQ ID NO: 14)

Das Codon, das für Asparaginsäure in Position 3 der Insulin B- Kette kodiert ist jeweils fett herausgestellt. Die Konstruktion wird wie ins Beispiel 1 beschrieben durchgeführt. Template ist DNA des Plasmides pINT3581. Richtige Plasmide erhalten die Bezeichnung pINT3588.

### Beispiel 10: Konstruktion des Plasmides pINT3589 kodierend für Glu (A5), His (A8), Gly (A21), Asp (B3)- Präproinsulin

Führt man die Reaktionen wie in Beispiel 9 beschrieben durch, verwendet aber in PCR1 und PCR2 DNA des Plasmides pINT3582 als Template, so gelangt man zu Plasmid pINT3589.

Das von dem Plasmid kodierte Prä - Proinsulin ist Vorstufe für die Verbindung YKL205-3, die nach Amidierung mit Argininamid entsteht und die folgende Struktur beschreibt:
Arg (A0), Glu (A5), His (A8), Gly (A21), Asp (B3), Arg (B31), Arg (B32) - NH₂-Humaninsulin

Das von dem Plasmid kodierte Prä - Proinsulin ist Vorstufe für die Verbindung YKL205-3b, die nach Amidierung mit Lysinamid entsteht und die folgende Struktur beschreibt:
Arg (A0), Glu (A5), His (A8), Gly (A21), Asp (B3), Arg (B31), Lys (B32) - NH₂-Humaninsulin

### Beispiel 11: Konstruktion des Plasmides pINT3590 kodierend für His (A8), Glu (A15), Gly (A21), Asp (B3) - Präproinsulin

Führt man die Reaktionen wie in Beispiel 9 beschrieben durch, verwendet aber in PCR1 und PCR2 DNA des Plasmides pINT3585 als Template, so gelangt man zu Plasmid pINT3590. Das von dem Plasmid kodierte Prä - Proinsulin ist Vorstufe für die Verbindung YKL205-4, die nach Amidierung mit Argininamid entsteht und die folgende Struktur beschreibt:
Arg (A0), His (A8), Glu (A15), Gly (A21), Asp (B3), Arg (B31), Arg (B32) - NH₂-Humaninsulin

Das von dem Plasmid kodierte Prä - Proinsulin ist Vorstufe für die Verbindung YKL205-4b, die nach Amidierung mit Lysinamid entsteht und die folgende Struktur beschreibt:
Arg (A0), His (A8), Glu (A15), Gly (A21), Asp (B3), Arg (B31), Lys (B32) - NH₂-Humaninsulin

### Beispiel 12: Konstruktion des Plasmides pINT3591 kodierend für His (A8), Asp (A18), Gly (A21), Asp (B3)- Präproinsulin

Führt man die Reaktionen wie in Beispiel 9 beschrieben durch, verwendet aber in PCR1 und PCR2 DNA des Plasmides pINT3586 als Template, so gelangt man zu Plasmid pINT3591. Das von dem Plasmid kodierte Prä - Proinsulin ist Vorstufe für die Verbindung YKL205-5, die nach Amidierung mit Argininamid entsteht und folgende Struktur beschreibt:
Arg (A0), His (A8), Asp (A18), Gly (A21), Asp (B3), Arg (B31), Arg (B32) - NH₂-Humaninsulin

Das von dem Plasmid kodierte Prä - Proinsulin ist Vorstufe für die Verbindung YKL205-5b, die nach Amidierung mit Lysinamid entsteht und die folgende Struktur beschreibt:
Arg (A0), His (A8), Asp (A18), Gly (A21), Asp (B3), Arg (B31), Lys (B32) - NH₂-Humaninsulin

### Beispiel 13: Konstruktion des Plasmides pINT3592 kodierend für His (A8), Gly (A21), Asp (B3)- Glu (B4) - Präproinsulin

Die Konstruktion erfolgt wie in Beispiel 1 und 2 beschrieben über 3 Polymerase Kettenreaktionen. Das Produkt der dritten Reaktion wird nach Nco1/ Sal1 Spaltung in die Nco1 / Sal1 geöffnete pINT91d Vektor DNA insertiert. Verwendet werden die Primer Tir und pint3580_glya21rev. Zwei weitere Primer werden synthetisiert:
pint3581_Db3_Eb4f
5'- GCACGATTTGTG**GACGA**GCACCTGTGCGGCTC -3' (SEQ ID NO: 15)
pint3581_Db3_Eb4rev
5'- CGCACAGG TG**CTCG**TCCA CAAATCGTGC CGAATTTC -3' (SEQ ID NO: 16)

Das Codon, das für Asparaginsäure in Position 3 und Glutaminsäure in Position 4 der Insulin B- Kette kodiert, ist jeweils fett herausgestellt Die Konstruktion wird wie in Beispiel 1 beschrieben durchgeführt. Template ist DNA des Plasmides pINT3581. Richtige Plasmide erhalten die Bezeichnung pINT3592. Das von dem Plasmid kodierte Prä - Proinsulin ist Vorstufe für die Verbindung YKL205-6, die nach Amidierung mit Argininamid entsteht und die folgende Struktur beschreibt:
Arg (A0), His (A8), Gly (A21), Asp (B3), Glu (B4), Arg (B31), Arg (B32) - NH₂-Humaninsulin

Das von dem Plasmid kodierte Prä - Proinsulin ist Vorstufe für die Verbindung YKL205-6b, die nach Amidierung mit Lysinamid entsteht und die folgende Struktur beschreibt:
Arg (A0), His (A8), Gly (A21), Asp (B3), Glu (B4), Arg (B31), Lys (B32) - NH₂-Humaninsulin

### Beispiel 14: Konstruktion des Plasmides pINT3593 kodierend für His (A8), Gly (A21), Glu (B4) - Präproinsulin

Die Konstruktion erfolgt wie in Beispiel 1 und 2 beschrieben über 3 Polymerase Kettenreaktionen. Das Produkt der dritten Reaktion wird nach Nco1/ Sal1 Spaltung in die Nco1 / Sal1 geöffnete pINT91d Vektor DNA insertiert. Verwendet werden die Primer Tir und pint3580_glya21rev. Zwei weitere Primer werden synthetisiert :
pint3581_Eb4f
5'- ACGATTTGTGAAC**GAG**CACCTGTGCGGCTC -3' (SEQ ID NO: 17)
pint3581_Eb4rev
5'- CGCACAGG TG**CTC**GTTCA CAAATCGTGC CGAATTTC -3' (SEQ ID NO: 18)

Das Codon, das für Glutaminsäure in Position 4 der Insulin B- Kette kodiert, ist fett herausgestellt. Die Konstruktion wird wie ins Beispiel 1 beschrieben durchgeführt. Template ist DNA des Plasmides pINT3581. Richtige Plasmide erhalten die Bezeichnung pINT3593.

### Beispiel 15: Konstruktion des Plasmides pINT3594 kodierend für Glu (A5), His (A8), Gly (A21), Glu (B4) - Präproinsulin.

Führt man die Reaktionen wie in Beispiel 9 beschrieben durch, verwendet aber in PCR1 und PCR2 DNA des Plasmides pINT3582 als Template, so gelangt man zu Plasmid pINT3594.

Das Proinsulin ist Vorstufe für die Verbindung YKL205-7, die nach Amidierung mit Argininamid entsteht und die folgende Struktur beschreibt:
Arg (A0), Glu (A5), His (A8), Gly (A21), Glu (B4), Arg (B31), Arg (B32) - NH₂-Humaninsulin.

Das Proinsulin ist Vorstufe für die Verbindung YKL205-7b, die nach Amidierung mit Lysinamid entsteht und die folgende Struktur beschreibt:
Arg (A0), Glu (A5), His (A8), Gly (A21), Glu (B4), Arg (B31), Lys (B32) - NH₂-Humaninsulin

### Beispiel 16: Konstruktion des Plasmides pINT3595 kodierend für His (A8), Glu (A15), Gly (A21), Glu (B4) - Präproinsulin.

Führt man die Reaktionen wie in Beispiel 9 beschrieben durch, verwendet aber in PCR1 und PCR2. DNA des Plasmides pINT3585 als Template, so gelangt man zu Plasmid pINT3595. Das von dem Plasmid kodierte Prä - Proinsulin ist Vorstufe für die Verbindung YKL205-8, die nach Amidierung mit Argininamid entsteht und die folgende Struktur beschreibt:
Arg (A0), His (A8), Glu (A15), Gly (A21), Glu (B4), Arg (B31), Arg (B32) - NH₂-Humaninsulin

Das von dem Plasmid kodierte Prä - Proinsulin ist Vorstufe für die Verbindung YKL205-8b, die nach Amidierung mit Lysinamid entsteht und die folgende Struktur beschreibt:
Arg (A0), His (A8), Glu (A15), Gly (A21), Glu (B4), Arg (B31), Lys (B32) - NH₂-Humaninsulin

### Beispiel 17: Konstruktion des Plasmides pINT3596 kodierend für His (A8), Asp (A18), Gly (A21), Glu (B4)- Präproinsulin

Führt man die Reaktionen wie in Beispiel 9 beschrieben durch, verwendet aber in PCR1 und PCR2 DNA des Plasmides pINT3586 als Template, so gelangt man zu Plasmid pINT3596. Das von dem Plasmid kodierte Prä - Proinsulin ist Vorstufe für die Verbindung YKL205-9, die nach Amidierung mit Argininamid entsteht und die folgende Struktur beschreibt:
Arg (A0), His (A8), Asp (A18), Gly (A21), Glu (B4), Arg (B31), Arg (B32) - NH₂-Humaninsulin

Das von dem Plasmid kodierte Prä - Proinsulin ist Vorstufe für die Verbindung YKL205-9b, die nach Amidierung mit Lysinamid entsteht und die folgende Struktur beschreibt:
Arg (A0), His (A8), Asp (A18), Gly (A21), Glu (B4), Arg (B31), Lys (B32) - NH₂-Humaninsulin

### Beispiel 18: Konstruktion des Plasmides pINT3597 kodierend für His (A8), Gly (A21), Glu (B0) -Präproinsulin

Die Konstruktion erfolgt über 2 Polymerase Kettenreaktionen. Verwendet wird der Primer pint3580_glya21rev. Zwei weitere Primer werden synthetisiert:
pint3581_Eb0f1
5'- CAACAGGAA ATTCGGCACG A**GAG**TTTGTG AACCAGCACC TGTG-3' (SEQ
ID NO: 19)
pint3581_Eb01f2
5'- TATCGA CCAT GG CAACAACA TCAACAGGAA ATTCGGCACG A**GAG**-3' (SEQ ID NO: 20)

Dabei überlappen sich die beiden Primer partial. Pint3581_Eb0f2 enthält eine NcoI-Erkennungssequenz. Diese ist unterstrichen dargestellt. Das Codon, das für Glutaminsäure in Position 0 am Anfang B- Kette kodiert, ist jeweils fett herausgestellt. Template für PCR1 ist DNA des Plasmides pINT3581.

PCR1 wird mit dem Primerpaar pint3581_Eb-1f2 / pint3580_glya21rev. Template für PCR2 ist das Produkt aus PCR1. PCR2 wird mit dem Primerpaar pint3581_Eb-1f2 / pint3580_glya21rev durchgeführt. Das Produkt aus PCR2 übereckt die vollständige Präproinsulinsequenz. Das Produkt der zweiten Reaktion wird nach Nco1/ Sal1 Spaltung in die Nco1 / Sal1 geöffnete pINT91d Vektor DNA insertiert. Richtige Plasmide erhalten die Bezeichnung pINT3597. Ersetzt man das Codon für Glutaminsäure in Position B0 durch das Codon von Asparaginsäure und folgt dem Beispiel, so gelangt man zu Plasmiden, die in Position B0 anstelle von Glutaminsäure Asparaginsäure tragen.

### Beispiel 19: Konstruktion des Plasmides pINT3598 kodierend für Glu (A5), His (A8), Gly (A21), Glu (B0) - Präproinsulin

Führt man die Reaktionen wie in Beispiel 18 beschrieben durch, verwendet aber in PCR1 DNA des Plasmides pINT3582 als Template, so gelangt man zu Plasmid pINT3598. Das von dem Plasmid kodierte Prä - Proinsulin ist Vorstufe für die Verbindung YKL205-10, die nach Amidierung mit Argininamid entsteht und die folgende Struktur beschreibt:
Arg (A0), Glu (A5), His (A8), Gly (A21), Glu (B0), Arg (B31), Arg (B32) - NH₂-Humaninsulin

Das von dem Plasmid kodierte Prä - Proinsulin ist Vorstufe für die Verbindung YKL205-10b, die nach Amidierung mit Lysinamid entsteht und die folgende Struktur beschreibt:
Arg (A0), Glu (A5), His (A8), Gly (A21), Glu (B0), Arg (B31), Lys (B32) - NH₂-Humaninsulin

### Beispiel 20: Konstruktion des Plasmides pINT3599 kodierend für His (A8), Glu (A15), Gly (A21), Glu (B0) - Präproinsulin

Führt man die Reaktionen wie in Beispiel 18 beschrieben durch, verwendet aber in PCR1 DNA des Plasmides pINT3585 als Template, so gelangt man zu Plasmid pINT3599. Das von dem Plasmid kodierte Prä - Proinsulin ist Vorstufe für die Verbindung YKL205-11, die nach Amidierung mit Argininamid entsteht und die folgende Struktur beschreibt:
Arg (A0), His (A8), Glu (A15), Gly (A21), Glu (B0), Arg (B31), Arg (B32) - NH₂-Humaninsulin

Das von dem Plasmid kodierte Prä - Proinsulin ist Vorstufe für die Verbindung YKL205-11b, die nach Amidierung mit Lysinamid entsteht und die folgende Struktur beschreibt:
Arg (A0), His (A8), Glu (A15), Gly (A21), Glu (B0), Arg (B31), Lys (B32) - NH₂-Humaninsulin

### Beispiel 21: Konstruktion des Plasmides pINT3600 kodierend für His (A8), Asp (A18), Gly (A21), Glu (B0) - Präproinsulin

Führt man die Reaktionen wie in Beispiel 18 beschrieben durch, verwendet aber in PCR1 DNA des Plasmides pINT3586 als Template, so gelangt man zu Plasmid pINT3600. Das von dem Plasmid kodierte Prä - Proinsulin ist Vorstufe für die Verbindung YKL205-12, die nach Amidierung mit Argininamid entsteht und die folgende Struktur beschreibt:
Arg (A0), His (A8), Asp (A18), Gly (A21), Glu (B0), Arg (B31), Arg (B32) - NH₂-Humaninsulin

Das von dem Plasmid kodierte Prä - Proinsulin ist Vorstufe für die Verbindung YKL205-12b, die nach Amidierung mit Lysinamid entsteht und die folgende Struktur beschreibt:
Arg (A0), His (A8), Asp (A18), Gly (A21), Glu (B0), Arg (B31), Lys (B32) - NH₂-Humaninsulin

### Beispiel 22: Konstruktion des Plasmides pINT3601 kodierend für His (A8), Gly (A21), Asp (B1) - Präproinsulin

Die Konstruktion erfolgt über 2 Polymerase Kettenreaktionen. Verwendet wird der Primer pint3580_glya21rev. Zwei weitere Primer werden synthetisiert:
pint3581_Db1f1
5'-CAACAGGAA ATTCGGCACG **AGAC**GTG AACCAGCACC TGTGCG-3' (SEQ ID NO: 21)
pint3581_Db1f2
5'-TATCGA CCAT GG CAACAACA TCAACAGGAA ATTCGGCACG A**GAC**-3' (SEQ ID NO: 22)

Dabei überlappen sich die beiden Primer partial. Pint3581_Db-1f2 enthält eine NcoI - Erkennungssequenz. Diese ist unterstrichen dargestellt. Das Codon, das für Asparaginsäure in Position 1 der B- Kette kodiert, ist jeweils fett herausgestellt. Template für PCR1 ist DNA des Plasmides pINT3581. PCR1 wird mit dem Primerpaar pint3581_Db1f1 / pint3580_glya21rev durchgeführt. Template für PCR2 ist das Produkt aus PCR1. PCR2 wird mit dem Primerpaar pint3581_Db1f2 / pint3580_glya21rev durchgeführt. Das Produkt aus PCR2 überdeckt die vollständige Präproinsulinsequenz. Das Produkt der zweiten Reaktion wird nach Nco1/ Sal1 Spaltung in die Nco1 / Sal1 geöffnete pINT91d Vektor DNA insertiert. Richtige Plasmide erhalten die Bezeichnung pINT3601.

### Beispiel 23: Konstruktion des Plasmides pINT3602 kodierend für Glu (A5), His (A8), Gly (A21), Asp (B1) - Präproinsulin

Führt man die Reaktionen wie in Beispiel 22 beschrieben durch, verwendet aber in PCR1 DNA des Plasmides pINT3582 als Template, so gelangt man zu Plasmid pINT3602. Das von dem Plasmid kodierte Prä - Proinsulin ist Vorstufe für die Verbindung YKL205-13, die nach Amidierung mit Argininamid entsteht und die folgende Struktur beschreibt:
Arg (A0), Glu (A5), His (A8), Gly (A21), Asp (B1), Arg (B31), Arg (B32) - NH₂-Humaninsulin

Das von dem Plasmid kodierte Prä - Proinsulin ist Vorstufe für die Verbindung YKL205-13b, die nach Amidierung mit Lysinamid entsteht und die folgende Struktur beschreibt:
Arg (A0), Glu (A5), His (A8), Gly (A21), Asp (B1), Arg (B31), Lys (B32) - NH₂-Humaninsulin

### Beispiel 24: Konstruktion des Plasmides pINT3603 kodierend für His (A8), Glu (A15), Gly (A21), Asp (B1) - Präproinsulin

Führt man die Reaktionen wie in Beispiel 22 beschrieben durch, verwendet aber in PCR1 DNA des Plasmides pINT3585 als Template, so gelangt man zu Plasmid pINT3603. Das von dem Plasmid kodierte Prä - Proinsulin ist Vorstufe für die Verbindung YKL205-14, die nach Amidierung mit Argininamid entsteht und die folgende Struktur beschreibt:
Arg (A0), His (A8), Glu (A15), Gly (A21), Asp (B1), Arg (B31), Arg (B32) - NH₂-Humaninsulin

Das von dem Plasmid kodierte Prä - Proinsulin ist Vorstufe für die Verbindung YKL205-14b, die nach Amidierung mit Lysinamid entsteht und die folgende Struktur beschreibt:
Arg (A0), His (A8), Glu (A15), Gly (A21), Asp (B1), Arg (B31), Lys (B32) - NH₂-Humaninsulin

### Beispiel 25: Konstruktion des Plasmides pINT3604 kodierend für His (A8), Asp (A18), Gly (A21), Asp (B1) - Präproinsulin

Führt man die Reaktionen wie in Beispiel 22 beschrieben durch, verwendet aber in PCR1 DNA des Plasmides pINT3586 als Template, so gelangt man zu Plasmid pINT3604. Das von dem Plasmid kodierte Prä - Proinsulin ist Vorstufe für die Verbindung YKL205-15, die nach Amidierung mit Argininamid entsteht und die folgende Struktur beschreibt:
Arg (A0), His (A8), Asp (A18), Gly (A21), Asp (B1), Arg (B31), Arg (B32) - NH₂-Humaninsulin

Das von dem Plasmid kodierte Prä - Proinsulin ist Vorstufe für die Verbindung YKL205-15b, die nach Amidierung mit Lysinamid entsteht und die folgende Struktur beschreibt:
Arg (A0), His (A8), Asp (A18), Gly (A21), Asp (B1), Arg (B31), Lys (B32) - NH₂-Humaninsulin

### Beispiel 26: Konstruktion des Plasmides pINT3605 kodierend für His (A8), Gly (A21), Glu (B0), Asp (B1) - Präproinsulin

Die Konstruktion erfolgt über 2 Polymerase Kettenreaktionen. Verwendet wird der Primer pint3580_glya21rev und der in Beispiel 18 beschrieben Primer pint3581_Eb01f2. Synthetisiert wird der Primer pint3597_Db1f:
5'-CAACAGGAA ATTCGGCACG A**GAGGAC**GTG AACCAGCACC TGTGC-3' (SEQ ID NO: 23)

Das Codon, das für Glutaminsäure in Position 0 und das für Asparaginsäure jeweils am Anfang der B- Kette kodiert, ist jeweils fett herausgestellt. Template für PCR1 ist DNA des Plasmides pINT3597. PCR1 wird mit dem Primerpaar pint3597_Db1f/ pint3580_glya21rev. Template für PCR2 ist das Produkt aus PCR1. PCR2 wird mit dem Primerpaar pint3581_Eb1f2 / pint3580_glya21rev durchgeführt. Das Produkt aus PCR2 überdeckt die vollständige Präproinsulinsequenz. Das Produkt der zweiten Reaktion wird nach Nco1/ Sal1 Spaltung in die Nco1 / Sal1 geöffnete pINT91d Vektor DNA insertiert. Richtige Plasmide erhalten die Bezeichnung pINT3605. Das von dem Plasmid kodierte Prä - Proinsulin ist Vorstufe für die Verbindung YKL205-16, die nach Amidierung mit Argininamid entsteht und die folgende Struktur beschreibt:
Arg (A0), His (A8), Gly (A21), Glu (B0), Asp (B1), Arg (B31), Arg (B32) - NH₂-Humaninsulin

Das von dem Plasmid kodierte Prä - Proinsulin ist Vorstufe für die Verbindung YKL205-16b, die nach Amidierung mit Lysinamid entsteht und die folgende Struktur beschreibt:
Arg (A0), His (A8), Gly (A21), Glu (B0), Asp (B1), Arg (B31), Lys (B32) - NH₂-Humaninsulin

### Beispiel 27: Konstruktion des Plasmides pINT3606 kodierend für His (A8), Glu (A15), Asp (A18), Gly (A21), desThr (B30) - Präproinsulin

Die Konstruktion erfolgt wie in Beispiel 1 und 2 beschrieben über 3 Polymerase Kettenreaktionen. Verwendet werden die Primer Tir und pint3580_glya21rev. Zwei weitere Primer werden synthetisiert:
desB30f
5'-TTCTACACACCC**AAG**CGCGATGTTCCTCAGGTGG-3' (SEQ ID NO: 24)
desB30rev
5'-AGG AACATCGCGC TTGGGTGTGT AGAAGAAGC-3' (SEQ ID NO: 25)

Template für PCR1 und PCR2 ist DNA des Plasmides pINT3586. PCR1 wird mit dem Primerpaar desB30f / pint3580_glya21rev und PCR2 wird mit dem Primerpaar Tir / desB30rev Template durchgeführt. Als Template für PCR3 wird ein äquimolares Gemisch der Produkte aus PCR1 und PCR2 verwendet. Die Reaktion wird mit dem Primerpaar Tir/ pint3580_glya21rev durchgeführt. Das Produkt aus PCR3 überdeckt die vollständige Präproinsulinsequenz. Das Produkt der dritten Reaktion wird nach Nco1/ Sal1 Spaltung in die Nco1 / Sal1 geöffnete pINT91d Vektor DNA insertiert. Das von dem Plasmid kodierte Prä - Proinsulin ist Vorstufe für die Verbindung YKL205-17, die nach Amidierung mit Argininamid entsteht und die folgende Struktur beschreibt:
Arg (A0), His (A8), Glu (A15), Asp (A18), Gly (A21), Arg (B30), Arg (B31) - NH₂-Humaninsulin

Das von dem Plasmid kodierte Prä - Proinsulin ist Vorstufe für die Verbindung YKL205-17b, die nach Amidierung mit Lysinamid entsteht und die folgende Struktur beschreibt:
Arg (A0), His (A8), Glu (A15), Asp (A18), Gly (A21), Arg (B30), Lys (B31) - NH₂-Humaninsulin

### Beispiel 28: Expression der Proinsulinderivate

Die Expression wird entsprechend Beispiel 1 der Europäischen Patentanmeldung EP-A 1 222 207 durchgeführt.

### Beispiel 29: Faltung der Proinsulinderivate

Die Faltung erfolgt prinzipiell nach der in EP-A 0 668 282 beschriebenen Methode Beispiel 30: Enzymatische Prozessierung der gefalteten Präproinsulin zu der 2 - kettigen Arg(A0) - Insulinvorstufe, deren C - terminales B-Kettenende durch Lysin oder Arginin charakterisiert ist.

Die Enzymatische Prozessierung der gefalteten Präproinsulinvorstufe erfolgt wie z.B. in Beispiel 4 von WO91/03550 beschrieben. Als besonders vorteilhaft erweist sich dabei der Einsatz der in WO 2007/031187 A1 beschriebenen Trypsinvariante.

### Beispiel 31: Herstellung eines Arg (A0), His (A8), Gly (A21), Arg (B31), Arg (B32)-NH₂- Humaninsulins

Unabhängig von der Positionierung der zusätzlichen sauren Aminosäuren wird eine Standardreaktion wie folgt durchgeführt: 100 mg Arg (A0), Gly (A21), Arg (B31) - Insulinanalog werden in 0.95 ml Argininamidlösung (446 g/L) gelöst, 0.13 mL M Na-Acetatpuffer (pH 5.8) und 2 ml DMF zugegeben. Die Reaktionsmischung wird auf 12° C gekühlt und durch Zugabe von 0.094 ml Trypsin (0.075 mg, Roche Diagnostics) gestartet. Nach 8 h wird die Reaktion durch Zugabe von TFA bis pH 2.5 gestoppt und per HPLC analysiert. Es bildet sich >60 % . Arg (A0), Gly (A21), Arg (B31), Arg (B32) - NH₂- Humaninsulin. Nach Zusatz von Trypsininhibitorlösung erfolgt die Reinigung des amidierten Analogs in Analogie zu US 5,656,722.

Die Herstellung der entsprechenden Lysinamid - Verbindung erfolgt analog. Allerdings geht man von einer wässrigen Lysinamid Stammlösung aus, die 366g/L Lysinamid gelöst enthält.

### Beispiel 32: Formulierung der amidierten Indulinderivate

Um die erfindungsgemäßen Insulinderivate auf ihre biologisch, pharmakologischen und physikalisch chemischen Eigenschaften zu testen, wurde von den Verbindungen wie folgt eine Lösung hergestellt: Das erfindungsgemäße Insulinderivat wurde mit einer Zielkonzentration von 240 ± 5 µM in 1 mM Salzsäure mit 80 µg/mL Zink (als Zinkchlorid) aufgelöst.

Als Lösungsmedium wurden folgende Zusammensetzungen verwendet:
a) 1 mM Salzsäure
b) 1 mM Salzsäure, 5 µg/mL Zink (als Zinkchlorid oder Salzsäure zugesetzt)
c) 1 mM Salzsäure, 10 µg/mL Zink (als Zinkchlorid oder Salzsäure zugesetzt)
d) 1 mM Salzsäure, 15 µg/mL Zink (als Zinkchlorid oder Salzsäure zugesetzt)
e) 1 mM Salzsäure, 30 µg/mL Zink (als Zinkchlorid oder Salzsäure zugesetzt)
f) 1 mM Salzsäure, 80 µg/mL Zink (als Zinkchlorid oder Salzsäure zugesetzt)
g) 1 mM Salzsäure, 120 µg/mL Zink (als Zinkchlorid oder Salzsäure zugesetzt)

Hierzu wurde von dem gefriergetrockneten Material zunächst eine um etwa 30% höhere Menge als aufgrund des Molekulargewichts und der angestrebten Konzentration benötigt eingewogen. Danach wurde die vorliegende Konzentration mittels analytischer HPLC bestimmt und die Lösung anschließend auf das zur Erreichung der Zielkonzentration erforderliche Volumen mit 5 mM Salzsäure mit 80 µg/mL Zink aufgefüllt. Falls erforderlich, wurde dabei der pH-Wert auf 3,5 ± 0,1 nachjustiert. Nach der endgültigen Analyse durch HPLC zur Absicherung der Zielkonzentration von 240 ± 5 µM wurde die fertige Lösung mittels einer Spritze mit einem 0,2 µm Filtervorsatz in ein mit einem Septum und einer Bördelkappe verschlossenes steriles Fläschchen überführt. Für die kurzfristige, einmalige Testung der erfindungsgemäßen Insulinderivate wurde keine Optimierung der Formulierungen, z.B. hinsichtlich eines Zusatzes isotonischer Agentien, Konservierungsmittel oder Puffersubstanzen, vorgenommen.

### Beispiel 33: Evaluierung der blutzuckersenkenden Wirkung von neuen Insulinanaloga in der Ratte

Die blutzuckersenkende Wirkung von ausgewählten neuen Insulinanaloga wird in männlichen, gesunden, normoglykämischen Wistarratten geprüft. Männlichen Ratten wird eine Dosis von 9 nmol/kg eines Insulinanalogons subkutan injiziert. Unmittelbar vor der Injektion des Insulinanalogons und in regelmäßigen Abständen bis zu acht Stunden nach der Injektion werden den Tieren Blutproben entnommen und darin der Blutzuckergehalt bestimmt. Das Experiment zeigt deutlich (vgl. Fig. 1), dass das eingesetzte erfindungsgemäße Insulinanalogon zu einem deutlich verzögerten Wirkeintritt und einer längeren, gleichmäßigen Wirkdauer führt.

### Beispiel 34: Evaluierung der blutzuckersenkenden Wirkung von neuen Insulinanaloga im Hund

Die blutzuckersenkende Wirkung von ausgewählten neuen Insulinanaloga wird in männlichen, gesunden, normoglykämischen Beaglehunden geprüft. Männlichen Tieren wird eine Dosis von 6 nmol/kg eines Insulinanalogons subkutan injiziert. Unmittelbar vor der Injektion des Insulinanalogons und in regelmäßigen Abständen bis zu achtundvierzig Stunden nach der Injektion werden den Tieren Blutproben entnommen und darin der Blutzuckergehalt bestimmt. Das Experiment zeigt deutlich (vgl. Fig. 2), dass das eingesetzte erfindungsgemäße Insulinanalogon zu einem deutlich verzögerten Wirkeintritt und einer längeren, gleichmäßigen Wirkdauer führt.

### Beispiel 35: Evaluierung der blutzuckersenkenden Wirkung am Hund bei zweifach erhöhter Dosis

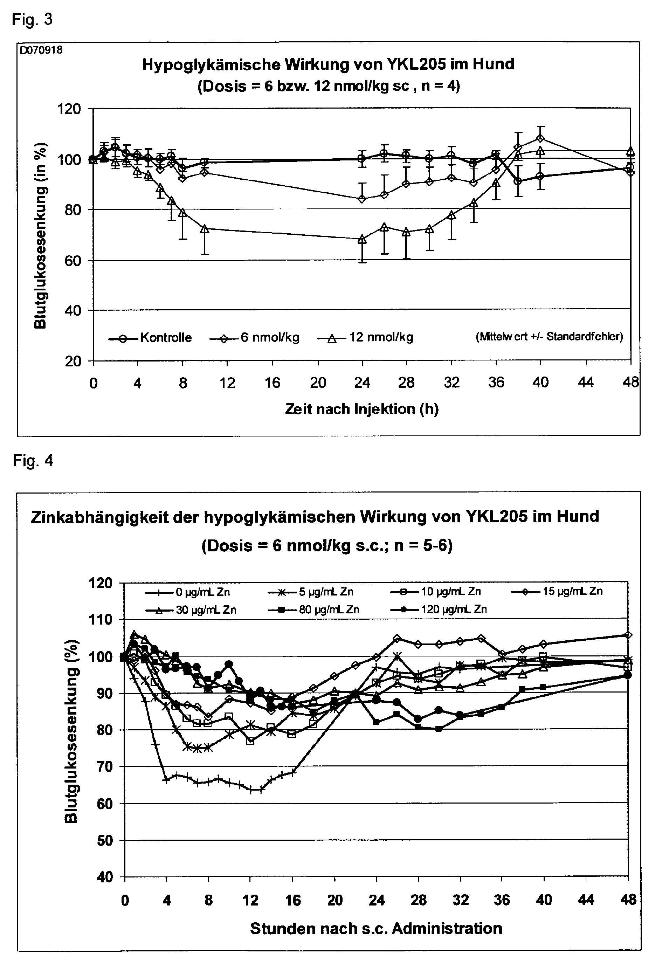

Die blutzuckersenkende Wirkung von ausgewählten neuen Insulinanaloga wird in männlichen, gesunden, normoglykämischen Beaglehunden geprüft. Männlichen Tieren wird eine Dosis von 6 nmol/kg und 12nmol/kg eines Insulinanalogons subkutan injiziert. Unmittelbar vor der Injektion des Insulinanalogons und in regelmäßigen Abständen bis zu achtundvierzig Stunden nach der Injektion werden den Tieren Blutproben entnommen und darin der Blutzuckergehalt bestimmt.
Das Experiment zeigt deutlich (vgl. Fig. 3), dass das eingesetzte erfindungsgemäße Insulinanalogon dosisabhängig wirkt, dass aber trotz zweifach erhöhter Dosis der Wirkungsverlauf flach verläuft, d.h. kein ausgeprägter Tiefpunkt (Nadir) beobachtet wird. Daraus lässt sich ableiten, dass die erfindungsgemäßen Insuline im Vergleich zu bekannten Verzögerungsinsulinen zu deutlich weniger hypoglykämischen Ereignissen führen.

### Beispiel 36: Evaluierung der blutzuckersenkenden Wirkung am Hund bei verschiedenen Zinkkonzentrationen in der Formulierung

Die Experimente wurden, wie in Beispiel 35 beschrieben, durchgeführt. Figur 4 zeigt das Ergebnis. Danach lässt sich die Zeit - Wirkungskurve des erfindungsgemäßen Insulinanalogons durch den Gehalt an Zinkionen in der Formulierung bei gleicher Insulinkonzentration in der Weise beeinflussen, dass man bei null oder geringem Zinkgehalt einen schnellen Wirkungseintritt beobachtet und die Wirkung über 24 Stunden anhält, während bei höherem Zinkgehalt ein flacher Wirkungseintritt beobachtet wird und die Insulinwirkung deutlich länger als 24 Stunden anhält.

## Patentansprüche

1. Insulinanalogon der Formel I wobei
A0 Arg;
A5 Asp, Gln oder Glu;
A15 Asp, Glu oder Gln;
A18 Asp, Glu oder Asn;
B-1 Asp, Glu oder eine Aminogruppe;
B0 Asp, Glu oder eine chemische Bindung;
B1 Asp, Glu oder Phe;
B2 Asp, Glu oder Val;
B3 Asp, Glu oder Asn;
B4 Asp, Glu oder Gln;
B29 Lys;
B30 Thr;
B31 Arg oder Lys;
B32 Arg-NH₂ oder Lys-NH₂
entspricht, wobei zwei Aminosäurereste der Gruppe enthaltend A5, A15, A18, B-1, B0, B1, B2, B3 und B4 gleichzeitig und unabhängig voneinander Asp oder Glu entsprechen.

2. Insulinanalogon gemäß einem oder mehreren der vorstehenden Ansprüche, wobei A5 Glu entspricht.

3. Insulinanalogon gemäß einem oder mehreren der vorstehenden Ansprüche, wobei A15 Glu entspricht.

4. Insulinanalogon gemäß einem oder mehreren der vorstehenden Ansprüche, wobei A18 Asp entspricht.

5. Insulinanalogon gemäß einem oder mehreren der vorstehenden Ansprüche, wobei B-1 einer Aminogruppe entspricht.

6. Insulinanalogon gemäß einem oder mehreren der vorstehenden Ansprüche, wobei B0 Glu entspricht.

7. Insulinanalogon gemäß einem oder mehreren der vorstehenden Ansprüche, wobei B1 Asp entspricht.

8. Insulinanalogon gemäß einem oder mehreren der vorstehenden Ansprüche, wobei B2 Val entspricht.

9. Insulinanalogon gemäß einem oder mehreren der vorstehenden Ansprüche, wobei B3 Asp entspricht.

10. Insulinanalogon gemäß einem oder mehreren der vorstehenden Ansprüche, wobei B4 Glu entspricht.

11. Insulinanalogon gemäß einem oder mehreren der vorstehenden Ansprüche, ausgewählt aus einer Gruppe enthaltend:
Arg (A0), His (A8), Glu (A5), Asp (A18), Gly (A21), Arg (B31), Arg (B32) - NH₂ Humaninsulin,
Arg (A0), His (A8), Glu (A5), Asp (A18), Gly (A21), Arg (B31), Lys (B32) - NH₂ Humaninsulin,
Arg (A0), His (A8), Glu (A15), Asp (A18), Gly (A21), Arg (B31), Arg (B32) - NH₂ Humaninsulin,
Arg (A0), His (A8), Glu (A15), Asp (A18), Gly (A21), Arg (B31), Lys (B32) - NH₂ Humaninsulin,
Arg (A0), His (A8), Glu(A5), Glu (A15), Gly (A21), Arg (B31), Arg (B32) - NH₂ Humaninsulin,
Arg (A0), His (A8), Glu (A5), Glu (A15), Gly (A21), Arg (B31), Lys (B32) - NH₂ Humaninsulin,
Arg (A0), His(A8), Glu (A5), Gly (A21), Asp (B3), Arg (B31), Arg (B32) - NH₂ Humaninsulin,
Arg (A0), His(A8), Glu (A5), Gly (A21), Asp (B3), Arg (B31), Lys (B32) - NH₂ Humaninsulin,
Arg (A0), His (A8), Glu (A15), Gly (A21), Asp (B3), Arg (B31), Arg (B32) - NH₂ Humaninsulin,
Arg (A0), His (A8), Glu (A15), Gly (A21), Asp (B3), Arg (B31), Lys (B32) - NH₂ Humaninsulin,
Arg (A0), His (A8), Asp (A18), Gly (A21), Asp (B3), Arg (B31), Arg (B32) - NH₂ Humaninsulin,
Arg (A0), His (A8), Asp (A18), Gly (A21), Asp (B3), Arg (B31), Lys (B32) - NH₂ Humaninsulin,
Arg (A0), His(A8), Gly (A21), Asp (B3), Glu (B4), Arg (B31), Arg (B32) - NH₂ Humaninsulin,
Arg (A0), His (A8), Gly (A21), Asp (B3), Glu (B4), Arg (B31), Lys (B32) - NH₂ Humaninsulin,
Arg (A0), His (A8), Glu (A5), Gly (A21), Glu (B4), Arg (B31), Arg (B32) - NH₂ Humaninsulin,
Arg (A0), His (A8), Glu (A5), Gly (A21), Glu (B4), Arg (B31), Lys (B32) - NH₂ Humaninsulin,
Arg (A0), His (A8), Glu (A15), Gly (A21), Glu (B4), Arg (B31), Arg (B32) - NH₂ Humaninsulin,
Arg (A0), His (A8), Glu (A15), Gly (A21), Glu (B4), Arg (B31), Lys (B32) - NH₂ Humaninsulin,
Arg (A0), His (A8), Asp (A18), Gly (A21), Glu (B4), Arg (B31), Arg (B32) - NH₂ Humaninsulin,
Arg (A0), His (A8), Asp (A18), Gly (A21), Glu (B4), Arg (B31), Lys (B32) - NH₂ Humaninsulin,
Arg (A0), His (A8), Glu (A5), Gly (A21), Glu (B0), Arg (B31), Arg (B32) - NH₂ Humaninsulin,
Arg (A0), His (A8), Glu (A5), Gly (A21), Glu (B0), Arg (B31), Lys (B32) - NH₂ Humaninsulin,
Arg (A0), His (A8), Glu (A15), Gly (A21), Glu (B0), Arg (B31), Arg (B32) - NH₂ Humaninsulin,
Arg (A0), His (A8), Glu (A15), Gly (A21), Glu (B0), Arg (B31), Lys (B32) - NH₂ Humaninsulin,
Arg (A0), His (A8), Asp (A18), Gly (A21), Glu (B0), Arg (B31), Arg (B32) - NH₂ Humaninsulin,
Arg (A0), His (A8), Asp (A18),Gly (A21), Glu (B0), Arg (B31), Lys (B32) - NH₂ Humaninsulin,
Arg (A0), His (A8), Glu (A5), Gly (A21), Asp (B1), Arg (B31), Arg (B32) - NH₂ Humaninsulin,
Arg (A0), His (A8), Glu (A5), Gly (A21), Asp (B1), Arg (B31), Lys (B32) - NH₂ Humaninsulin,
Arg (A0), His (A8), Glu (A15), Gly (A21), Asp (B1), Arg (B31), Arg(B32) - NH₂ Humaninsulin,
Arg (A0), His (A8), Glu (A15), Gly (A21), Asp (B1), Arg (B31), Lys (B32) - NH₂ Humaninsulin,
Arg (A0), His (A8), Asp (A18), Gly (A21), Asp (B1), Arg (B31), Arg (B32) - NH₂ Humaninsulin,
Arg (A0), His (A8), Asp (A18), Gly (A21), Asp (B1), Arg (B31), Lys (B32) - NH₂ Humaninsulin,
Arg (A0), His (A8), Gly (A21), Glu (B0), Asp (B1), Arg (B31), Arg (B32) - NH₂ Humaninsulin,
Arg (A0), His (A8), Gly (A21), Glu (B0), Asp (B1), Arg (B31), Lys (B32) - NH₂ Humaninsulin,
Arg (A0), His (A8), Asp (A18), Gly (A21), Asp (B3), Arg (B30), Arg (B31) - NH₂ Humaninsulin,
Arg (A0), His (A8), Asp (A18), Gly (A21), Asp (B3), Arg (B30), Lys (B31) - NH₂ Humaninsulin.

12. Verfahren zur Herstellung eines Insulinanalogons gemäß einem der Ansprüche 1 bis 11, wobei ein Vorläufer des Insulinanalogons rekombinant hergestellt wird, der Vorläufer enzmatisch zu zwei -kettigem Insulin prozessiert wird und eine Kupplung mit Argininamid in Gegenwart eines Enzyms mit Trypsinaktivität durchgeführt wird, und das Insulinanalogon isoliert wird.

13. Verwendung eines Insulinanalogons gemäß einem der Ansprüche 1 bis 11 zur Herstellung eines Medikaments zur Behandlung von Diabetes Mellitus.

14. Verwendung eines Insulinanalogons gemäß einem der Ansprüche 1 bis 11 in einem Verfahren zur Herstellung eines Medikaments zur Behandlung von Diabetes MellitusTyp I oder Typ II oder zur therapeutischen Unterstützung der beta -Zellregeneration.

15. Arzneimittel enthaltend ein Insulinanalog gemäß einem der Ansprüche 1 bis 11 und/oder physiologisch verträgliche Salze davon.

16. Formulierung des Insulinanalogons gemäß einem der Ansprüche 1 bis 11, wobei die Formulierung in wässriger Form enthaltend das gelöste Insulinanalogon vorliegt.

17. Formulierung des Insulinanalogons gemäß einem der Ansprüche 1 bis 11, wobei die Formulierung in Form von Pulver vorliegt.

18. Formulierung gemäß Anspruch 17, wobei das Insulinanalogon gemäß einem der Ansprüche 1 bis 11 in kristalliner und/oder amorpher Form vorhanden ist.

19. Formulierung des Insulinanalogons gemäß einem der Ansprüche 1 bis 11, wobei die Formulierung in Form einer Suspension vorliegt.

20. Formulierung des Insulinanalogons gemäß einem der Ansprüche 1 bis 11, wobei die Formulierung zusätzlich ein chemisches Chaperon enthält.

21. DNA kodierend für ein Prä-Proinsulin eines Insulinanalogons gemäß einem der Ansprüche 1 bis 11.

22. Vektor enthaltend eine DNA nach Anspruch 21.

23. Nicht-humaner Wirtsorganismus enthaltend eine DNA nach Anspruch 21 oder einen Vektor gemäß Anspruch 22.

24. Formulierung nach einem oder mehreren der Ansprüche 16 bis 20, bei der noch zusätzlich ein Glucagon-Like Peptide-1 (GLP1) oder ein Analogon oder Derivat davon, oder Exendin-3 bzw. -4 oder ein Analogon oder Derivat davon enthalten ist.

25. Formulierung nach Anspruch 24, bei dem zusätzlich Exendin-4 enthalten ist.

26. Formulierung gemäß Anspruch 24, bei dem ein Analogon von Exendin-4 ausgewählt wird aus einer Gruppe enthaltend
H-desPro³⁶-Exendin-4-Lys₆-NH₂,
H-des(Pro^{36,37})-Exendin-4-Lys₄-NH₂ und
H-des(Pro^{36,37})-Exendin-4-Lys₅-NH₂, oder ein pharmakologisch tolerierbares Salz davon.

27. Formulierung gemäß Anspruch 24, bei dem ein Analogon von Exendin-4 ausgewählt wird aus einer Gruppe enthaltend
desPro³⁶ [Asp²⁸]Exendin-4 (1-39),
desPro³⁶ [IsoAsp²⁸]Exendin-4 (1-39),
desPro³⁶ [Met(O)¹⁴, Asp²⁸]Exendin-4 (1-39),
desPro³⁶ [Met(O)¹⁴, IsoAsp²⁸]Exendin-4 (1-39),
desPro³⁶ [Trp(O₂)²⁵ Asp²⁸]Exendin-2 (1-39),
desPro³⁶ [Trp(O₂)²⁵, IsoAsp²⁸]Exendin-2 (1-39),
desPro³⁶ [Met(O)¹⁴Trp(O₂)²⁵, Asp²⁸]Exendin-4 (1-39) und
desPro³⁶ [Met(O)¹⁴Trp(O₂)²⁵, IsoAsp²⁸]Exendin-4 (1-39), oder ein pharmakologisch tolerierbares Salz davon.

28. Formulierung gemäß Anspruch 27, bei denen an die C-Termini der Analoga von Exendin-4 das Peptid -Lys₆-NH₂ angefügt ist.

29. Formulierung gemäß Anspruch 24, bei dem ein Analogon von Exendin-4 ausgewählt wird aus einer Gruppe enthaltend
H-(Lys)₆- des Pro³⁶ [Asp²⁸]Exendin-4(1-39)-Lys₆-NH₂
des Asp²⁸Pro³⁶, Pro³⁷, Pro₃₈ Exendin-4(1-39) -NH₂,
H-(Lys)₆- des Pro³⁶, Pro³⁷, Pro³⁸ [Asp²⁸]Exendin-4(1-39)-NH₂,
H-Asn-(Glu)₅ des Pro³⁶, Pro³⁷, Pro³⁸ [Asp²⁸]Exendin-4(1-39)-NH₂,
des Pro³⁶, Pro³⁷, Pro³⁸ [Asp²⁸]Exendin-4(1-39)-(Lys)₆-NH₂,
H-(Lys)₆- des Pro³⁶, Pro³⁷, Pro³⁸ [Asp²⁸]Exendin-4(1-39)-(Lys)₆-NH₂,
H-Asn-(Glu)₅- des Pro³⁶, Pro³⁷, Pro³⁸ [Asp²⁸]Exendin-4(1-39)-(Lys)₆-NH₂,
H-(Lys)₆- des Pro³⁶ [Trp(O₂)²⁵, Asp²⁸]Exendin-4(1-39)-Lys₆-NH₂,
H- des Asp²⁸ Pro³⁶, Pro³⁷, Pro³⁸ [Trp(O₂)²⁵]Exendin-4(1-39-NH₂,
H-(Lys)₆- des Pro³⁶, Pro³⁷, Pro³⁸ [Trp(O₂)²⁵, Asp²⁸]Exendin-4(1-39) -NH₂,
H-Asn-(Glu)₅- des Pro³⁶, Pro³⁷, Pro³⁸ [Trp(O₂)²⁵, Asp²⁸]Exendin-4(1-39) -NH₂,
des Pro³⁶, Pro³⁷, Pro³⁸ [Trp(O₂)²⁵, Asp²⁸]Exendin-4(1-39)-(Lys)₆-NH₂,
H-(Lys)₆- des Pro³⁶, Pro³⁷, Pro³⁸ [Trp(O₂)²⁵, Asp²⁸]Exendin-4(1-39)-(Lys)₆-NH₂,
H-Asn-(Glu)5- des Pro³⁶, Pro³⁷, Pro³⁸ [Trp(O₂)²⁵, Asp²⁸]Exendin-4(1-39)-(Lys)₆-NH₂,
H-(Lys)₆- des Pro³⁶ [Met(O)¹⁴, Asp²⁸]Exendin-4(1-39)-Lys₆-NH₂,
des Met(O)¹⁴ Asp²⁸ Pro³⁶, Pro³⁷, Pro³⁸ Exendin-4(1-39) -NH₂,
H-(Lys)₆- des Pro³⁶, Pro³⁷, Pro³⁸ [Met(O)¹⁴, Asp²⁸]Exendin-4(1-39) -NH₂,
H-Asn-(Glu)5- des Pro³⁶, Pro³⁷, Pro³⁸ [Met(O)¹⁴, Asp²⁸] Exendin-4(1-39) -NH₂,
des Pro³⁶, Pro³⁷, Pro³⁸ [Met(O)¹⁴, Asp²⁸]Exendin-4(1-39)-(Lys)₆-NH₂,
H-(Lys)₆- des Pro³⁶, Pro³⁷, Pro³⁸ [Met(O)¹⁴, Asp²⁸]Exendin-4(1-39)-Lys₆-NH₂,
H-Asn-(Glu)₅ des Pro³⁶, Pro³⁷, Pro³⁸ [Met(O)¹⁴, Asp²⁸] Exendin-4(1-39)-(Lys)6-NH2,
H-(Lys)₆- des Pro³⁶ [Met(O)¹⁴, Trp(O₂)²⁵, Asp²⁸]Exendin-4(1-39)-Lys₆-NH₂,
des Asp²⁸ Pro³⁶, Pro³⁷, Pro³⁸ [Met(O)¹⁴, Trp(O₂)²⁵]Exendin-4(1-39)-NH₂,
H-(Lys)₆- des Pro³⁶, Pro³⁷, Pro³⁸ [Met(O)¹⁴, Trp(O₂)²⁵, Asp²⁸]Exendin-4(1-39) -NH₂,
H-Asn-(Glu)5- des Pro³⁶, Pro³⁷, Pro³⁸ [Met(O)¹⁴, Asp²⁸] Exendin-4(1-39) -NH₂,
des Pro³⁶, Pro³⁷, Pro³⁸ [Met(O)¹⁴, Trp(O₂)²⁵, Asp²⁸]Exendin-4(1-39)-(Lys)₆-NH₂,
H-(Lys)₆- des Pro³⁶, Pro³⁷, Pro³⁸ [Met(O)¹⁴, Trp(O₂)²⁵, Asp²⁸]Exendin-4(1-39)-(Lys)₆-NH₂,
H-Asn-(Glu)₅- des Pro³⁶, Pro³⁷, Pro³⁸ [Met(O)¹⁴, Trp(O₂)²⁵, Asp²⁸]Exendin-4(1-39)-(Lys)₆-NH₂, oder ein pharmakologisch tolerierbares Salz davon.

30. Formulierung nach Anspruch 24, bei dem zusätzlich Arg³⁴, Lys²⁶ (N^{ε}(γ-glutamyl(N^{α}-hexadecanoyl))) GLP-1 (7-37) [liraglutide] oder ein pharmakologisch tolerierbares Salz davon enthalten ist.

31. Wässrige Formulierung des Insulinanalogons gemäß einem der Ansprüche 1 bis 11, die kein oder weniger als 15 µg/ml Zink enthält.

32. Wässrige Formulierung des Insulinanalogons gemäß einem der Ansprüche 1 bis 11, die kein oder weniger als 15 µg/ml bis 2 mg/ml Zink enthält.

33. Formulierung gemäß Anspruch 32, wobei 200 µg/ml Zink enthalten sind.

## Claims

1. An insulin analogue of the formula I where
A0 corresponds to Arg;
A5 corresponds to Asp, Gln or Glu;
A15 corresponds to Asp, Glu or Gln;
A18 corresponds to Asp, Glu or Asn;
B-1 corresponds to Asp, Glu or an amino group;
B0 corresponds to Asp, Glu or a chemical bond;
B1 corresponds to Asp, Glu or Phe;
B2 corresponds to Asp, Glu or Val;
B3 corresponds to Asp, Glu or Asn;
B4 corresponds to Asp, Glu or Gln;
B29 corresponds to Lys;
B30 corresponds to Thr;
B31 corresponds to Arg or Lys;
B32 corresponds to Arg-NH₂ or Lys- NH₂,
where two amino acid residues of the group comprising A5, A15, A18, B-1, B0, B1, B2, B3 and B4 correspond simultaneously and independently of one another to Asp or Glu.

2. The insulin analogue as claimed in one or more of the preceding claims, where A5 corresponds to Glu.

3. The insulin analogue as claimed in one or more of the preceding claims, where A15 corresponds to Glu.

4. The insulin analogue as claimed in one or more of the preceding claims, where A18 corresponds to Asp.

5. The insulin analogue as claimed in one or more of the preceding claims, where B-1 corresponds to an amino group.

6. The insulin analogue as claimed in one or more of the preceding claims, where B0 corresponds to Glu.

7. The insulin analogue as claimed in one or more of the preceding claims, where B1 corresponds to Asp.

8. The insulin analogue as claimed in one or more of the preceding claims, where B2 corresponds to Val.

9. The insulin analogue as claimed in one or more of the preceding claims, where B3 corresponds to Asp.

10. The insulin analogue as claimed in one or more of the preceding claims, where B4 corresponds to Glu.

11. The insulin analogue as claimed in one or more of the preceding claims, selected from a group comprising:
Arg (A0), His (A8), Glu (A5), Asp (A18), Gly (A21), Arg (B31), Arg (B32) - NH₂ human insulin,
Arg (A0), His (A8), Glu (A5), Asp (A18), Gly (A21), Arg (B31), Lys (B32) - NH₂ human insulin,
Arg (A0), His (A8), Glu (A15), Asp (A18), Gly (A21), Arg (B31), Arg (B32) - NH₂ human insulin,
Arg (A0), His (A8), Glu (A15), Asp (A18), Gly (A21), Arg (B31), Lys (B32) - NH₂ human insulin,
Arg (A0), His (A8), Glu(A5), Glu (A15), Gly (A21), Arg (B31), Arg (B32) - NH₂ human insulin,
Arg (A0), His (A8), Glu (A5), Glu (A15), Gly (A21), Arg (B31), Lys (B32) - NH₂ human insulin,
Arg (A0), His(A8), Glu (A5), Gly (A21), Asp (B3), Arg (B31), Arg (B32) - NH₂ human insulin,
Arg (A0), His(A8), Glu (A5), Gly (A21), Asp (B3), Arg (B31), Lys (B32) - NH₂ human insulin,
Arg (A0), His (A8), Glu (A15), Gly (A21), Asp (B3), Arg (B31), Arg (B32) - NH₂ human insulin,
Arg (A0), His (A8), Glu (A15), Gly (A21), Asp (B3), Arg (B31), Lys (B32) - NH₂ human insulin,
Arg (A0), His (A8), Asp (A18), Gly (A21), Asp (B3), Arg (B31), Arg (B32) - NH₂ human insulin,
Arg (A0), His (A8), Asp (A18), Gly (A21), Asp (B3), Arg (B31), Lys (B32) - NH₂ human insulin,
Arg (A0), His(A8), Gly (A21), Asp (B3), Glu (B4), Arg (B31), Arg (B32) - NH₂ human insulin,
Arg (A0), His (A8), Gly (A21), Asp (B3), Glu (B4), Arg (B31), Lys (B32) - NH₂ human insulin,
Arg (A0), His (A8), Glu (A5), Gly (A21), Glu (B4), Arg (B31), Arg (B32) - NH₂ human insulin,
Arg (A0), His (A8), Glu (A5), Gly (A21), Glu (B4), Arg (B31), Lys (B32) - NH₂ human insulin,
Arg (A0), His (A8), Glu (A15), Gly (A21), Glu (B4), Arg (B31), Arg (B32) - NH₂ human insulin,
Arg (A0), His (A8), Glu (A15), Gly (A21), Glu (B4), Arg (B31), Lys (B32) - NH₂ human insulin,
Arg (A0), His (A8), Asp (A18), Gly (A21), Glu (B4), Arg (B31), Arg (B32) - NH₂ human insulin,
Arg (A0), His (A8), Asp (A18), Gly (A21), Glu (B4), Arg (B31), Lys (B32) - NH₂ human insulin,
Arg (A0), His (A8), Glu (A5), Gly (A21), Glu (B0), Arg (B31), Arg (B32) - NH₂ human insulin,
Arg (A0), His (A8), Glu (A5), Gly (A21), Glu (B0), Arg (B31), Lys (B32) - NH₂ human insulin,
Arg (A0), His (A8), Glu (A15), Gly (A21), Glu (B0), Arg (B31), Arg (B32) - NH₂ human insulin,
Arg (A0), His (A8), Glu (A15), Gly (A21), Glu (B0), Arg (B31), Lys (B32) - NH₂ human insulin,
Arg (A0), His (A8), Asp (A18), Gly (A21), Glu (B0), Arg (B31), Arg (B32) - NH₂ human insulin,
Arg (A0), His (A8), Asp (A18) ,Gly (A21), Glu (B0), Arg (B31), Lys (B32) - NH₂ human insulin,
Arg (A0), His (A8), Glu (A5), Gly (A21), Asp (B1), Arg (B31), Arg (B32) - NH₂ human insulin,
Arg (A0), His (A8), Glu (A5), Gly (A21), Asp (B1), Arg (B31), Lys (B32) - NH₂ human insulin,
Arg (A0), His (A8), Glu (A15), Gly (A21), Asp (B1), Arg (B31), Arg(B32) - NH₂ human insulin,
Arg (A0), His (A8), Glu (A15), Gly (A21), Asp (B1), Arg (B31), Lys (B32) - NH₂ human insulin,
Arg (A0), His (A8), Asp (A18), Gly (A21), Asp (B1), Arg (B31), Arg (B32) - NH₂ human insulin,
Arg (A0), His (A8), Asp (A18), Gly (A21), Asp (B1), Arg (B31), Lys (B32) - NH₂ human insulin,
Arg (A0), His (A8), Gly (A21), Glu (B0), Asp (B1), Arg (B31), Arg (B32) - NH₂ human insulin,
Arg (A0), His (A8), Gly (A21), Glu (B0), Asp (B1), Arg (B31), Lys (B32) - NH₂ human insulin,
Arg (A0), His (A8), Asp (A18), Gly (A21), Asp (B3), Arg (B30), Arg (B31) - NH₂ human insulin,
Arg (A0), His (A8), Asp (A18), Gly (A21), Asp (B3), Arg (B30), Lys (B31) - NH₂ human insulin.

12. A process for preparing an insulin analogue as claimed in any of claims 1 to 11, where a precursor of the insulin analogue is prepared recombinantly, the precursor is processed enzymatically to two-chain insulin, and a coupling with argininamide is carried out in the presence of an enzyme having trypsin activity, and the insulin analogue is isolated.

13. The use of an insulin analogue as claimed in any of claims 1 to 11 for the manufacture of a medicament for treating diabetes mellitus.

14. The use of an insulin analogue as claimed in one of claims 1 to 11 in a process for the manufacture of a medicament for the treatment of diabetes mellitus of type I or type II or for therapeutically assisting beta cell regeneration.

15. A pharmaceutical comprising an insulin analogue as claimed in any of claims 1 to 11 and/or physiologically acceptable salts thereof.

16. A formulation of the insulin analogue as claimed in any of claims 1 to 11, where the formulation is in aqueous form comprising the dissolved insulin analogue.

17. A formulation of the insulin analogue as claimed in any one of claims 1 to 11, where the formulation is in the form of powder.

18. The formulation as claimed in claim 17, where the insulin analogue as claimed in any of claims 1 to 11 is present in crystalline and/or amorphous form.

19. A formulation of the insulin analogue as claimed in any of claims 1 to 11, where the formulation is in the form of a suspension.

20. A formulation of the insulin analogue as claimed in any of claims 1 to 11, where the formulation additionally comprises a chemical chaperone.

21. DNA coding for a preproinsulin of an insulin analogue as claimed in any of claims 1 to 11.

22. A vector comprising a DNA as claimed in claim 21.

23. A non-human host organism comprising a DNA as claimed in claim 21 or a vector as claimed in claim 22.

24. The formulation as claimed in one or more of claims 16 to 20, which additionally comprises a glucagon-like peptide-1 (GLP1) or an analogue or derivative thereof, or exendin-3 or -4 or an analogue or derivative thereof.

25. The formulation as claimed in claim 24, which additionally comprises exendin-4.

26. The formulation as claimed in claim 24, wherein an analogue of exendin-4 is selected from a group comprising
H-desPro³⁶-exendin-4-Lys₆-NH₂,
H-des(Pro^{36,37})-exendin-4-Lys₄-NH₂ and
H-des(Pro^{36,37})-exendin-4-Lys₅-NH₂,
or a pharmacologically tolerable salt thereof.

27. The formulation as claimed in claim 24, wherein an analogue of exendin-4 is selected from a group comprising
desPro³⁶ [Asp²⁸]exendin-4 (1-39),
desPro³⁶ [IsoAsp²⁸]exendin-4 (1-39),
desPro³⁶ [Met(O)¹⁴, Asp²⁸]exendin-4 (1-39),
desPro³⁶ [Met(O)¹⁴, IsoAsp²⁸]exendin-4 (1-39),
desPro³⁶ [Trp(O₂)²⁵, Asp²⁸]exendin-2 (1-39),
desPro³⁶ [Trp(O₂)²⁵, IsoAsp²⁸]exendin-2 (1-39),
desPro³⁶ [Met(O)¹⁴Trp(O₂)²⁵, Asp²⁸]exendin-4 (1-39) and
desPro³⁶ [Met(O)¹⁴Trp(O₂)²⁵, IsoAsp²⁸]exendin-4 (1-39),
or a pharmacologically tolerable salt thereof.

28. The formulation as claimed in claim 27, where the peptide -Lys₆-NH₂ is attached to the C termini of the analogues of exendin-4.

29. The formulation as claimed in claim 24, where an analogue of exendin-4 is selected from a group comprising
H-(Lys)₆- des Pro³⁶ [Asp²⁸]exendin-4(1-39)-Lys₆-NH₂
des Asp²⁸Pro³⁶, Pro³⁷, Pro³⁸ exendin-4(1-39) -NH₂,
H-(Lys)₆- des Pro³⁶, Pro³⁷, Pro³⁸ [Asp²⁸]exendin-4(1-39) -NH₂,
H-Asn-(Glu)₅ des Pro³⁶, Pro³⁷, Pro³⁸ [Asp²⁸]exendin-4(1-39) -NH₂,
des Pro³⁶, Pro³⁷, Pro³⁸ [Asp²⁸]exendin-4(1-39)-(Lys)₆-NH₂,
H-(Lys)₆- des Pro³⁶, Pro³⁷, Pro³⁸ [Asp²⁸]exendin-4(1-39)-(Lys)₆₋NH₂,
H-Asn-(Glu)₅- des Pro³⁶, Pro³⁷, Pro³⁸ [Asp²⁸]exendin-4(1-39)-(Lys)₆-NH₂,
H-(Lys)₆- des Pro³⁶ [Trp(O₂)²⁵, Asp²⁸]exendin-4(1-39)-Lys₆-NH₂,
H- des Asp²⁸ Pro³⁶, Pro³⁷, Pro³⁸ [Trp(O₂)²⁵]exendin-4(1-39)-NH₂,
H-(Lys)₆- des Pro³⁶, Pro³⁷, Pro³⁸ [Trp(O₂)²⁵, Asp²⁸]exendin-4(1-39) -NH₂,
H-Asn-(Glu)₅- des Pro³⁶, Pro³⁷, Pro³⁸ [Trp(O₂)²⁵, Asp²⁸]exendin-4(1-39) -NH₂,
des Pro³⁶, Pro³⁷, Pro³⁸ [Trp(O₂)²⁵, Asp²⁸]exendin-4(1-39)-(Lys)₆-NH₂,
H-(Lys)₆- des Pro³⁶, Pro³⁷, Pro³⁸ [Tᵣp(O₂)²⁵, Asp²⁸]exendin-4(1-39)-(Lys)6-NH₂,
H-Asn-(Glu)₅- des Pro³⁶, Pro³⁷, Pro³⁸ [Trp(O₂)²⁵, Asp²⁸]exendin-4(1-39)-(Lys)₆-NH₂,
H-(Lys)₆- des Pro³⁶ [Met(O)¹⁴, Asp²⁸]exendin-4(1-39)-Lys₆-NH₂,
des Met(O)¹⁴ Asp²⁸ Pro³⁶, Pro³⁷, Pro³⁸ exendin-4(1-39) -NH₂,
H-(Lys)₆- des Pro³⁶, Pro³⁷, Pro³⁸ [Met(O)¹⁴, Asp²⁸]exendin-4(1-39) -NH₂,
H-Asn-(Glu)₅- des Pro³⁶, Pro³⁷, Pro³⁸ [Met(O)¹⁴, Asp²⁸] exendin-4(1-39) -NH₂,
des Pro³⁶, Pro³⁷, Pro³⁸ [Met(O)¹⁴, Asp²⁸]exendin-4(1-39)-(Lys)₆-NH₂,
H-(Lys)₆- des Pro³⁶, Pro³⁷, Pro³⁸ [Met(O)¹⁴, Asp²⁸]exendin-4(1-39)-Lys₆-NH₂,
H-Asn-(Glu)₅ des Pro³⁶, Pro³⁷, Pro³⁸ [Met(O)¹⁴, Asp²⁸]exendin-4(1-39)-(Lys)₆-NH₂,
H-(Lys)₆- des Pro³⁶ [Met(O)¹⁴, Trp(O₂)²⁵, Asp²⁸]exendin-4(1-39)-Lys₆-NH₂,
des Asp²⁸ Pro³⁶, Pro³⁷, Pro³⁸ [Met(O)¹⁴, Trp(O₂)²⁵]exendin-4(1-39) -NH₂,
H-(Lys)₆- des Pro³⁶, Pro³⁷, Pro³⁸ [Met(O)¹⁴, Trp(O₂)²⁵, Asp²⁸]exendin-4(1-39) -NH₂,
H-Asn-(Glu)₅- des Pro³⁶, Pro³⁷, Pro³⁸ [Met(O)¹⁴, Asp²⁸] exendin-4(1-39) -NH₂,
des Pro³⁶, Pro³⁷, Pro³⁸ [Met(O)¹⁴, Trp(O₂)²⁵, Asp²⁸]exendin-4(1-39)-(Lys)₆-NH₂,
H-(Lys)₆- des Pro³⁶, Pro³⁷, Pro³⁸ [Met(O)¹⁴, Trp(O₂)²⁵, Asp²⁸]exendin-4(1-39)-(Lys)₆-NH₂,
H-Asn-(Glu)₅- des Pro³⁶, Pro³⁷, Pro³⁸ [Met(O)¹⁴, Trp(O₂)²⁵, Asp²⁸] exendin-4(1-39)-(Lys)₆-NH₂,
or a pharmacologically tolerable salt thereof.

30. The formulation as claimed in claim 24, which additionally comprises Arg³⁴, Lys²⁶ (N^{ε}(γ-glutamyl(N^{α}-hexadecanoyl))) GLP-1 (7-37) [liraglutide] or a pharmacologically tolerable salt thereof.

31. An aqueous formulation of the insulin analogue as claimed in any of claims 1 to 11, which comprises no zinc or less than 15 µg/ml of zinc.

32. An aqueous formulation of the insulin analogue as claimed in any of claims 1 to 11, which comprises no zinc or less than 15 µg/ml to 2 mg/ml of zinc.

33. The formulation as claimed in claim 32, where the zinc content is 200 µg/ml.

## Revendications

1. Analogue d'insuline de formule I où
A0 correspond à Arg ;
A5 correspond à Asp, Gln ou Glu ;
A15 correspond à Asp, Glu ou Gln ;
A18 correspond à Asp, Glu ou Asn ;
B-1 correspond à Asp, Glu ou un groupe amino ;
B0 correspond à Asp, Glu ou une liaison chimique ;
B1 correspond à Asp, Glu ou Phe ;
B2 correspond à Asp, Glu ou Val ;
B3 correspond à Asp, Glu ou Asn ;
B4 correspond à Asp, Glu ou Gln ;
B29 correspond à Lys;
B30 correspond à Thr;
B31 correspond à Arg ou Lys;
B32 correspond à Arg-NH₂ ou Lys-NH₂,
où deux résidus d'acide aminé du groupe comprenant A5, A15, A18, B-1, B0, B1, B2, B3 et B4 correspondent simultanément et indépendamment l'un de l'autre à Asp ou Glu.

2. Analogue d'insuline selon une ou plusieurs des revendications précédentes, où A5 correspond à Glu.

3. Analogue d'insuline selon une ou plusieurs des revendications précédentes, où A15 correspond à Glu.

4. Analogue d'insuline selon une ou plusieurs des revendications précédentes, où A18 correspond à Asp.

5. Analogue d'insuline selon une ou plusieurs des revendications précédentes, où B-1 correspond à un groupe amino.

6. Analogue d'insuline selon une ou plusieurs des revendications précédentes, où B0 correspond à Glu.

7. Analogue d'insuline selon une ou plusieurs des revendications précédentes, où B1 correspond à Asp,

8. Analogue d'insuline selon une ou plusieurs des revendications précédentes, où B2 correspond à Val.

9. Analogue d'insuline selon une ou plusieurs des revendications précédentes, où B3 correspond à Asp.

10. Analogue d'insuline selon une ou plusieurs des revendications précédentes, où B4 correspond à Glu.

11. Analogue d'insuline selon une ou plusieurs des revendications précédentes, choisi dans un groupe comprenant :
Arg (A0), His (A8), Glu (A5), Asp (A18), Gly (A21), Arg (B31), Arg (B32) - NH₂ insuline humaine,
Arg (A0), His (A8), Glu (A5), Asp (A18), Gly (A21), Arg (B31), Lys (B32) - NH₂ insuline humaine,
Arg (A0), His (A8), Glu (A15), Asp (A18), Gly (A21), Arg (B31), Arg (B32) - NH₂ insuline humaine,
Arg (A0), His (A8), Glu (A15), Asp (A18), Gly (A21), Arg (B31), Lys (B32) - NH₂ insuline humaine,
Arg (A0), His (A8), Glu(A5), Glu (A15), Gly (A21), Arg (B31), Arg (B32) - NH₂ insuline humaine,
Arg (A0), His (A8), Glu (A5), Glu (A15), Gly (A21), Arg (B31), Lys (B32) - NH₂ insuline humaine,
Arg (A0), His(A8), Glu (A5), Gly (A21), Asp (B3), Arg (B31), Arg (B32) - NH₂ insuline humaine,
Arg (A0), His(A8), Glu (A5), Gly (A21), Asp (B3), Arg (B31), Lys (B32) - NH₂ insuline humaine,
Arg (A0), His (A8), Glu (A15), Gly (A21), Asp (B3), Arg (B31), Arg (B32) - NH₂ insuline humaine,
Arg (A0), His (A8), Glu (A15), Gly (A21), Asp (B3), Arg (B31), Lys (B32) - NH₂ insuline humaine,
Arg (A0), His (A8), Asp (A18), Gly (A21), Asp (B3), Arg (B31), Arg (B32) - NH₂ insuline humaine,
Arg (A0), His (A8), Asp (A18), Gly (A21), Asp (B3), Arg (B31), Lys (B32) - NH₂ insuline humaine,
Arg (A0), His(A8), Gly (A21), Asp (B3), Glu (B4), Arg (B31), Arg (B32) - NH₂ insuline humaine,
Arg (A0), His (A8), Gly (A21), Asp (B3), Glu (B4), Arg (B31), Lys (B32) - NH₂ insuline humaine,
Arg (A0), His (A8), Glu (A5), Gly (A21), Glu (B4), Arg (B31), Arg (B32) - NH₂ insuline humaine,
Arg (A0), His (A8), Glu (A5), Gly (A21), Glu (B4), Arg (B31), Lys (B32) - NH₂ insuline humaine,
Arg (A0), His (A8), Glu (A15), Gly (A21), Glu (B4), Arg (B31), Arg (B32) - NH₂ insuline humaine,
Arg (A0), His (A8), Glu (A15), Gly (A21), Glu (B4), Arg (B31), Lys (B32) - NH₂ insuline humaine,
Arg (A0), His (A8), Asp (A18), Gly (A21), Glu (B4), Arg (B31), Arg (B32) - NH₂ insuline humaine,
Arg (A0), His (A8), Asp (A18), Gly (A21), Glu (B4), Arg (B31), Lys (B32) - NH₂ insuline humaine,
Arg (A0), His (A8), Glu (A5), Gly (A21), Glu (B0), Arg (B31), Arg (B32) - NH₂ insuline humaine,
Arg (A0), His (A8), Glu (A5), Gly (A21), Glu (B0), Arg (B31), Lys (B32) - NH₂ insuline humaine,
Arg (A0), His (A8), Glu (A15), Gly (A21), Glu (B0), Arg (B31), Arg (B32) - NH₂ insuline humaine,
Arg (A0), His (A8), Glu (A15), Gly (A21), Glu (B0), Arg (B31), Lys (B32) - NH₂ insuline humaine,
Arg (A0), His (A8), Asp (A18), Gly (A21), Glu (B0), Arg (B31), Arg (B32) - NH₂ insuline humaine,
Arg (A0), His (A8), Asp (A18) ,Gly (A21), Glu (B0), Arg (B31), Lys (B32) - NH₂ insuline humaine,
Arg (A0), His (A8), Glu (A5), Gly (A21), Asp (B1), Arg (B31), Arg (B32) - NH₂ insuline humaine,
Arg (A0), His (A8), Glu (A5), Gly (A21), Asp (B1), Arg (B31), Lys (B32) - NH₂ insuline humaine,
Arg (A0), His (A8), Glu (A15), Gly (A21), Asp (B1), Arg (B31), Arg(B32) - NH₂ insuline humaine,
Arg (A0), His (A8), Glu (A15), Gly (A21), Asp (B1), Arg (B31), Lys (B32) - NH₂ insuline humaine,
Arg (A0), His (A8), Asp (A18), Gly (A21), Asp (B1), Arg (B31), Arg (B32) - NH₂ insuline humaine,
Arg (A0), His (A8), Asp (A18), Gly (A21), Asp (B1), Arg (B31), Lys (B32) - NH₂ insuline humaine,
Arg (A0), His (A8), Gly (A21), Glu (B0), Asp (B1), Arg (B31), Arg (B32) - NH₂ insuline humaine,
Arg (A0), His (A8), Gly (A21), Glu (B0), Asp (B1), Arg (B31), Lys (B32) - NH₂ insuline humaine,
Arg (A0), His (A8), Asp (A18), Gly (A21), Asp (B3), Arg (B30), Arg (B31) - NH₂ insuline humaine,
Arg (A0), His (A8), Asp (A18), Gly (A21), Asp (B3), Arg (B30), Lys (B31) - NH₂ insuline humaine.

12. Procédé pour préparer un analogue d'insuline selon l'une quelconque des revendications 1 à 11, dans lequel un précurseur de l'analogue d'insuline est préparé de manière recombinante, le précurseur est traité de manière enzymatique en insuline bicaténaire, et un couplage avec l'argininamide est effectué en présence d'une enzyme ayant une activité trypsine, et l'analogue d'insuline est isolé.

13. Utilisation d'un analogue d'insuline selon l'une quelconque des revendications 1 à 11 pour la fabrication d'un médicament pour traiter le diabète sucré.

14. Utilisation d'un analogue d'insuline selon l'une quelconque des revendications 1 à 11 dans un procédé pour la fabrication d'un médicament pour le traitement du diabète sucré de type I ou de type II ou pour assister de manière thérapeutique la régénération des cellules bêta.

15. Agent pharmaceutique comprenant un analogue d'insuline selon l'une quelconque des revendications 1 à 11 et/ou des sels physiologiquement acceptables de celui-ci.

16. Formulation de l'analogue d'insuline selon l'une quelconque des revendications 1 à 11, la formulation étant sous forme aqueuse comprenant l'analogue d'insuline dissous.

17. Formulation de l'analogue d'insuline selon l'une quelconque des revendications 1 à 11, la formulation étant sous la forme de poudre.

18. Formulation selon la revendication 17, dans laquelle l'analogue d'insuline selon l'une quelconque des revendications 1 à 11 est présent sous forme cristalline et/ou amorphe.

19. Formulation de l'analogue d'insuline selon l'une quelconque des revendications 1 à 11, la formulation étant sous la forme de suspension.

20. Formulation de l'analogue d'insuline selon l'une quelconque des revendications 1 à 11, la formulation comprenant en outre un chaperon chimique.

21. ADN codant pour une préproinsuline d'un analogue d'insuline selon l'une quelconque des revendications 1 à 11.

22. Vecteur comprenant un ADN selon la revendication 21.

23. Organisme hôte non-humain comprenant un ADN selon la revendication 21 ou un vecteur selon la revendication 22.

24. Formulation selon une ou plusieurs des revendications 16 à 20, qui comprend en outre un glucagon-like peptide-1 (GLP1) ou un analogue ou dérivé de celui-ci, ou l'exendine-3 ou -4 ou un analogue ou dérivé de celle-ci.

25. Formulation selon la revendication 24, qui comprend en outre l'exendine-4.

26. Formulation selon la revendication 24, dans laquelle un analogue d'exendine-4 est choisi dans un groupe comprenant
H-desPro³⁶-exendine-4-Lys₆-NH₂,
H-des(Pro^{36,37})-exendine-4-Lys₄-NH₂ et
H-des(Pro^{36,37})-exendine-4-Lys₅-NH₂, ou un sel pharmacologiquement tolérable de ceux-ci.

27. Formulation selon la revendication 24, dans laquelle un analogue d'exendine-4 est choisi dans un groupe comprenant
desPro³⁶ [Asp²⁸]exendine-4 (1-39),
desPro³⁶ [IsoAsp²⁸]exendine-4 (1-39),
desPro³⁶ [Met(O)¹⁴, Asp²⁸]exendine-4 (1-39),
desPro³⁶ [Met(O)¹⁴, IsoAsp²⁸]exendine-4 (1-39),
desPro³⁶ [Trp(O₂)²⁵, Asp²⁸]exendine-2 (1-39),
desPro³⁶ [Trp(O₂)²⁵, IsoAsp²⁸]exendine-2 (1-39),
desPro³⁶ [Met(O)¹⁴Trp(O₂)²⁵, Asp²⁸]exendine-4 (1-39) et
desPro³⁶ [Met(O)¹⁴Trp(O₂)²⁵, IsoAsp²⁸]exendine-4 (1-39), ou un sel pharmacologiquement tolérable de ceux-ci.

28. Formulation selon la revendication 27, dans laquelle le peptide -Lys₆-NH₂ est lié aux terminaisons C des analogues d'exendine-4.

29. Formulation selon la revendication 24, dans laquelle un analogue d'exendine-4 est choisi dans un groupe comprenant
H-(Lys)₆-desPro³⁶ [Asp²⁸]exendine-4(1-39)-Lys₆-NH₂
des Asp²⁸Pro³⁶, Pro³⁷, Pro³⁸ exendine-4(1-39)-NH₂,
H-(Lys)₆-desPro³⁶, Pro³⁷, Pro³⁸ [Asp²⁸]exendine-4(1-39)-NH₂,
H-Asn-(Glu)₅ desPro³⁶, Pro³⁷, Pro³⁸ [Asp²⁸]exendine-4(1-39)-NH₂,
des Pro³⁶, Pro³⁷, Pro³⁸ [Asp²⁸]exendine-4(1-39)-(Lys)₆-NH₂,
H-(Lys)₆-desPro³⁶, Pro³⁷, Pro³⁸ [Asp²⁸]exendine-4(1-39)-(Lys)₆-NH₂,
H-Asn-(Glu)₅-desPro³⁶, Pro³⁷, Pro³⁸ [Asp²⁸]exendine-4(1-39)-(Lys)₆₋NH₂,
H-(Lyₛ)₆-desPro³⁶ [Trp(O₂)²⁵, Asp²⁸]exendine-4(1-39)-Lys₆-NH₂,
H₋desAsp²⁸ Pro³⁶, Pro³⁷, Pro³⁸ [Trp(O₂)²⁵]exendine-4(1-39)-NH₂,
H-(Lys)₆-desPro³⁶, Pro³⁷, Pro³⁸ [Trp(O₂)²⁵, Asp²⁸]exendine-4(1-39)-NH₂,
H-Asn-(Glu)₅-desPro³⁶, Pro³⁷, Pro³⁸ [Trp(O₂)²⁵, Asp²⁸]exendine-4(1-39)-NH₂,
des Pro³⁶, Pro³⁷, Pro³⁸ [Trp(O₂)²⁵, Asp²⁸]exendine-4(1-39)-(Lys)₆-NH₂,
H-(Lys)₆-desPro³⁶, Pro³⁷, Pro³⁸ [Trp(O₂)²⁵, Asp²⁸]exendine-4(1-39)-(Lys)₆-NH₂,
H-Asn-(Glu)₅-desPro³⁶, Pro³⁷, Pro³⁸ [Trp(O₂)²⁵, Asp²⁸]exendine-4(1-39)-(Lys)₆-NH₂,
H-(Lys)₆-desPro³⁶ [Met(O)¹⁴, Asp²⁸]exendine-4(1-39)-Lys₆-NH₂,
des Met(O)¹⁴ Asp²⁸ Pro³⁶, Pro³⁷, Pro³⁸ exendine-4(1-39)-NH₂,
H-(Lys)₆-desPro³⁶, Pro³⁷, Pro³⁸ [Met(O)¹⁴, Asp²⁸]exendine-4(1-39)-NH₂,
H-Asn-(Glu)5-desPro³⁶, Pro³⁷, Pro³⁸ [Met(O)¹⁴, Asp²⁸] exendine-4(1-39)-NH₂,
des Pro³⁶, Pro³⁷, Pro³⁸ [Met(O)¹⁴, Asp²⁸]exendine-4(1-39)-(Lys)₆-NH₂,
H-(Lys)₆-desPro³⁶, Pro³⁷, Pro³⁸ [Met(O)¹⁴, Asp²⁸]exendine-4(1-39)-Lys₆-NH₂,
H-Asn-(Glu)₅ desPro³⁶, Pro³⁷, Pro³⁸ [Met(O)¹⁴, Asp²⁸]exendine-4(1-39)-(Lys)₆-NH₂,
H-(Lys)₆-desPro³⁶ [Met(O)¹⁴, Trp(O₂)²⁵, Asp²⁸]exendine-4(1-39)-Lys₆-NH₂,
des Asp²⁸ Pro³⁶, Pro³⁷, Pro³⁸ [Met(O)¹⁴, Trp(O₂)²⁵]exendine-4(1-39)-NH₂,
H-(Lys)₆-desPro³⁶, Pro³⁷, Pro³⁸ [Met(O)¹⁴, Trp(O₂)²⁵, Asp²⁸]exendine-4(1-39)-NH₂,
H-Asn-(Glu)₅-desPro³⁶, Pro³⁷, Pro³⁸ [Met(O)¹⁴, Asp²⁸]exendine-4(1-39)-NH₂,
des Pro³⁶, Pro³⁷, Pro³⁸ [Met(O)¹⁴, Trp(O₂)²⁵, Asp²⁸]exendine-4(1-39)-(Lys)₆-NH₂,
H-(Lys)₆-desPro³⁶, Pro³⁷, Pro³⁸ [Met(O)¹⁴, Trp(O₂)²⁵, Asp²⁸]exendine-4(1-39)-(Lys)₆-NH₂,
H-Asn-(Glu)₅-desPro³⁶, Pro³⁷, Pro³⁸ [Met(O)¹⁴, Trp(O₂)²⁵, Asp²⁸] exendine-4(1-39)-(Lys)₆-NH₂, ou un sel pharmacologiquement tolérable de ceux-ci.

30. Formulation selon la revendication 24, qui comprend en outre Arg³⁴, Lys²⁶ (N^{ε}(γ-glutamyl(N^{α}-hexadécanoyl)))-GLP-1 (7-37) [liraglutide] ou un sel pharmacologiquement tolérable de celui-ci.

31. Formulation aqueuse de l'analogue d'insuline selon l'une quelconque des revendications 1 à 11, qui ne comprend pas de zinc ou moins de 15 µg/ml de zinc.

32. Formulation aqueuse de l'analogue d'insuline selon l'une quelconque des revendications 1 à 11, qui ne comprend pas de zinc ou moins de 15 µg/ml à 2 mg/ml de zinc.

33. Formulation selon la revendication 32, dans laquelle la teneur en zinc est de 200 µg/ml.


### SEQUENCE LISTING

<110> Sanofi-Aventis Deutschland GmbH
<120> Neue Insulinderivate mit extrem verzögertem Zeit- / Wirkungsprofil
<130> DE2008/001
<140> 102008003568.8-43
   <141> 2008-01-09
<160> 25
<170> PatentIn version 3.3
<210> 1
   <211> 22
   <212> PRT
   <213> Artificial
<220>
   <223> A-Kette
<220>
   <221> MISC_FEATURE
   <222> (1)..(1)
   <223> Xaa ist Lys oder Arg
<220>
   <221> MISC_FEATURE
   <222> (6)..(6)
   <223> Xaa ist Asp, Gln oder Glu
<220>
   <221> MISC_FEATURE
   <222> (16)..(16)
   <223> Xaa ist Asp, Glu oder Gln
<220>
   <221> MISC_FEATURE
   <222> (19)..(19)
   <223> Xaa ist Asp, Glu oder Asn
<400> 1
   Xaa Gly Ile Val Glu Xaa Cys Cys His Ser Ile Cys Ser Leu Tyr Xaa
   1               5                   10                  15
   Leu Glu Xaa Tyr Cys Gly
               20
<210> 2
   <211> 34
   <212> PRT
   <213> Artificial
<220>
   <223> B-Kette
<220>
   <221> MISC_FEATURE
   <222> (1)..(1)
   <223> Xaa ist Asp, Glu oder eine Aminogruppe
<220>
   <221> MISC_FEATURE
   <222> (2)..(2)
   <223> Xaa ist Asp, Glu oder eine chemische Bindung
<220>
   <221> MISC_FEATURE
   <222> (3)..(3)
   <223> Xaa ist Asp, Glu oder Phe
<220>
   <221> MISC_FEATURE
   <222> (4)..(4)
   <223> Xaa ist Asp, Glu oder Val
<220>
   <221> MISC_FEATURE
   <222> (5)..(5)
   <223> Xaa ist Asp, Glu oder Asn
<220>
   <221> MISC_FEATURE
   <222> (6)..(6)
   <223> Xaa ist Asp, Glu oder Gln
<220>
   <221> MISC_FEATURE
   <222> (31)..(31)
   <223> Xaa ist Lys oder eine chemische Bindung
<220>
   <221> MISC_FEATURE
   <222> (32)..(32)
   <223> Xaa ist Thr oder eine chemische Bindung
<220>
   <221> MISC_FEATURE
   <222> (33)..(33)
   <223> Xaa ist Arg, Lys oder eine chemische Bindung
<220>
   <221> MISC_FEATURE
   <222> (34)..(34)
   <223> Xaa ist Arg-Amid, Lys-Amid oder eine Aminogruppe
<400> 2
   Xaa Xaa Xaa Xaa Xaa Xaa His Leu Cys Gly Ser His Leu Val Glu Ala
   1               5                   10                  15
   Leu Tyr Leu Val Cys Gly Glu Arg Gly Phe Phe Tyr Thr Pro Xaa Xaa
               20                  25                  30
   Xaa Xaa
<210> 3
   <211> 38
   <212> DNA
   <213> Artificial
<220>
   <223> pint3580_glya21rev
<400> 3
   caaaggtcga ctattagccg cagtagttct ccagctgg 38
<210> 4
   <211> 31
   <212> DNA
   <213> Artificial
<220>
   <223> arg_cjuncf
<400> 4
   gtccctgcag cgtcgcggca tcgtggagca g 31
<210> 5
   <211> 36
   <212> DNA
   <213> Artificial
<220>
   <223> arg_cjunc_rev
<400> 5
   ccacgatgcc gcgacgctgc agggacccct ccagcg 36
<210> 6
   <211> 32
   <212> DNA
   <213> Artificial
<220>
   <223> pint3580_Ha8f
<400> 6
   agcagtgctg ccacagcatc tgctccctct ac 32
<210> 7
   <211> 33
   <212> DNA
   <213> Artificial
<220>
   <223> pint3580_Ha8rev
<400> 7
   gagcagatgc tgtggcagca ctgctccacg atg 33
<210> 8
   <211> 31
   <212> DNA
   <213> Artificial
<220>
   <223> pint3581_Ea5f
<400> 8
   gcatcgtgga ggagtgctgc cacagcatct g 31
<210> 9
   <211> 32
   <212> DNA
   <213> Artificial
<220>
   <223> pint3581_Ea5rev
<400> 9
   ctgtggcagc actcctccac gatgccgcga cg 32
<210> 10
   <211> 47
   <212> DNA
   <213> Artificial
<220>
   <223> pint3580_Da18rev
<400> 10
   caaaggtcga ctattagccg cagtagtcct ccagctggta gagggag 47
<210> 11
   <211> 56
   <212> DNA
   <213> Artificial
<220>
   <223> pint3580_Ea15rev
<400> 11
   caaaggtcga ctattagccg cagtagttct ccagctcgta gagggagcag atgctg 56
<210> 12
   <211> 56
   <212> DNA
   <213> Artificial
<220>
   <223> pint3585_Ea15_Da18rev
<400> 12
   caaaggtcga ctattagccg cagtagtcct ccagctcgta gagggagcag atgctg 56
<210> 13
   <211> 30
   <212> DNA
   <213> Artificial
<220>
   <223> pint3581_Db3f
<400> 13
   gcacgatttg tggaccagca cctgtgcggc 30
<210> 14
   <211> 34
   <212> DNA
   <213> Artificial
<220>
   <223> pint3581_Db3rev
<400> 14
   cacaggtgct ggtccacaaa tcgtgccgaa tttc 34
<210> 15
   <211> 32
   <212> DNA
   <213> Artificial
<220>
   <223> pint3581_Db3_Eb4f
<400> 15
   gcacgatttg tggacgagca cctgtgcggc tc 32
<210> 16
   <211> 36
   <212> DNA
   <213> Artificial
<220>
   <223> pint3581_Db3_Eb4rev
<400> 16
   cgcacaggtg ctcgtccaca aatcgtgccg aatttc 36
<210> 17
   <211> 30
   <212> DNA
   <213> Artificial
<220>
   <223> pint3581_Eb4f
<400> 17
   acgatttgtg aacgagcacc tgtgcggctc 30
<210> 18
   <211> 36
   <212> DNA
   <213> Artificial
<220>
   <223> pint3581_Eb4rev
<400> 18
   cgcacaggtg ctcgttcaca aatcgtgccg aatttc 36
<210> 19
   <211> 43
   <212> DNA
   <213> Artificial
<220>
   <223> pint3581_Eb0f1
<400> 19
   caacaggaaa ttcggcacga gagtttgtga accagcacct gtg 43
<210> 20
   <211> 44
   <212> DNA
   <213> Artificial
<220>
   <223> pint3581_Eb01f2
<400> 20
   tatcgaccat ggcaacaaca tcaacaggaa attcggcacg agag 44
<210> 21
   <211> 42
   <212> DNA
   <213> Artificial
<220>
   <223> pint3581_Db1f1
<400> 21
   caacaggaaa ttcggcacga gacgtgaacc agcacctgtg cg 42
<210> 22
   <211> 44
   <212> DNA
   <213> Artificial
<220>
   <223> pint3581_Db1f2
<400> 22
   tatcgaccat ggcaacaaca tcaacaggaa attcggcacg agac 44
<210> 23
   <211> 44
   <212> DNA
   <213> Artificial
<220>
   <223> pint3597_Db1f
<400> 23
   caacaggaaa ttcggcacga gaggacgtga accagcacct gtgc 44
<210> 24
   <211> 34
   <212> DNA
   <213> Artificial
<220>
   <223> desB30f
<400> 24
   ttctacacac ccaagcgcga tgttcctcag gtgg 34
<210> 25
   <211> 32
   <212> DNA
   <213> Artificial
<220>
   <223> desB30rev
<400> 25
   aggaacatcg cgcttgggtg tgtagaagaa gc 32